# EUROPEAN PATENT APPLICATION

(11) **EP 4 733 383 A1**
(43) Date of publication of application: **29.04.2026**
(21) Application number: 24826011.9
(22) Date of filing: 21.06.2024
(51) Int. Cl.: C12N 1/00, C12N 5/0789

(54) **CULTURE MEDIUM AND BLOOD CELL CLONE, AND METHOD FOR PRODUCING SAME**

(30) Priority: 21.06.2023 JP 2023102048
(71) Applicant: Japan Institute for Health Security, Tokyo 162-8655 (JP); KANAGAWA INSTITUTE OF INDUSTRIAL SCIENCE AND TECHNOLOGY, Ebina-shi Kanagawa 243-0435 (JP)
(72) Inventor: TAKUBO, Keiyo, Tokyo 162-8655 (JP); WATANUKI, Shintaro, Tokyo 162-8655 (JP)
(74) Representative: Cabinet Becker et Associés
(86) International application number: PCT/JP2024/022563
(87) International publication number: WO 2024/262610

(57) **Abstract**

A culture medium for expanding hematopoietic cells capable of cell division, comprising a compound (I) of the following formula or a pharmaceutically acceptable salt thereof: 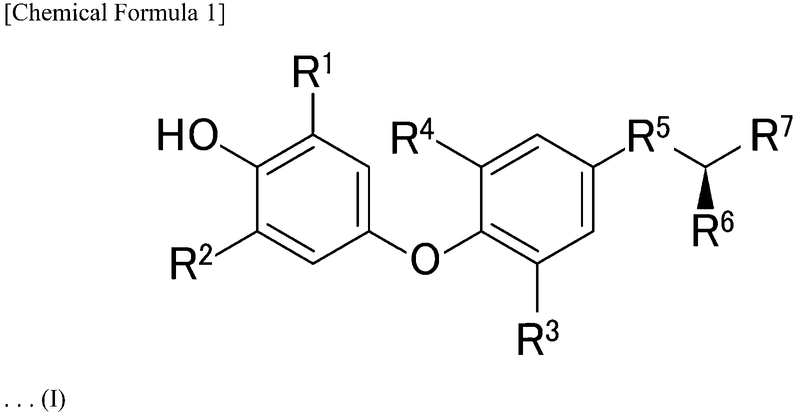 wherein R¹, R², and R³ are each hydrogen, straight-chain or branched C₁-C₄ alkyl, bromo, or iodo; R⁴ is straight-chain or branched C₁-C₄ alkyl, bromo, or iodo; R⁵ is oxygen, methylene, or a direct bond; R⁶ is hydrogen or -NH₂; and R⁷ is hydrogen or -COOH, provided that R⁶ and R⁷ are not both hydrogen.

## Description

### TECHNICAL FIELD

The present invention relates to a culture medium, a clone of hematopoietic stem cells (HSC) and other hematopoietic cells capable of cell division, and a method for producing the same, particularly one utilizing thyroxine, a thyroid hormone, and its metabolites.

### BACKGROUND ART

Patent Literature 1 discloses a cell culture kit. In this kit, triiodo-L-thyronine, a type of thyroid hormone or its metabolite, is selected as a labile factor (Claim 9). Furthermore, the labile factor does not induce differentiation of the cells during culture. Additionally, hematopoietic progenitor cells are selected as the cells (Claim 23).

Patent Literature 2 discloses a method for preparing a T-cell composition from stem cells. In this method, a cell aggregate containing stromal cells and stem cells is cultured in a medium (Claim 1). The medium contains triiodo-L-thyronine (Claim 12).

According to Non-Patent Literature 1, the biological activity of thyroid hormones is primarily mediated by triiodo-L-thyronine (T3) binding to its nuclear receptor. This nuclear receptor acts as a ligand-induced transcription factor, regulating the expression of target genes in a hormone-dependent manner. Furthermore, Kruppel-like factor 9 (KLF9) is primarily downregulated in hypothyroidism mutants. KLF9 is directly regulated by thyroid hormones. Moreover, knockdown of klf9 in zebrafish embryos inhibits hematopoietic development, including erythrocyte maturation and T lymphocyte formation.

As described above, Patent Literature 1 discloses a technique for culturing hematopoietic progenitor cells using triiodo-L-thyronine. Patent Literature 2 discloses a technique for inducing differentiation into T lymphocytes using triiodo-L-thyronine. Non-Patent Literature 1 discloses a technique for inhibiting T lymphocyte formation by knocking down the receptor for triiodo-L-thyronine. None of these technologies expand hematopoietic stem cells while suppressing their differentiation.

Turning to compounds other than triiodo-L-thyronine, mouse hematopoietic stem cells can be expanded using polyvinyl alcohol (PVA) (Non-Patent Literature 2), and human hematopoietic stem cells can be expanded using UM171, Soluplus (trademark, polyvinyl caprolactam-polyvinyl acetate-polyethylene glycol graft copolymer, PCL-PVAc-PEG), cell-permeable phosphorylated peptide 740Y-P, and butyzamide (Non-Patent Literature 3 and Non-Patent Literature 4).

Turning to techniques for suppressing hematopoietic stem cell differentiation, Patent Literature 3 and Non-Patent Literature 5 are cited. Conventionally, hematopoietic stem cells proliferate and differentiate rapidly in vitro. However, when conditions of very low cytokine concentration, hypoxia, and very high fatty acid concentration are combined, both proliferation and differentiation are suppressed. This allows hematopoietic stem cells to be maintained in a quiescent state suitable for transplantation by suppressing their proliferation and differentiation for one month.

### CITATION LIST

### PATENT LITERATURE

Patent Literature 1: JP2014-533113A
Patent Literature 2: JP2018-533364A
Patent Literature 3: JP2019-201599A

### NON-PATENT LITERATURE

Non-Patent Literature 1: Ying Zhang, Yuanyuan Xue, Chunwei Cao, Jiaojiao Huang, Qianlong Hong, Tang Hai, Qitao Jia, Xianlong Wang, Guosong Qin, Jing Yao, Xiao Wang, Qiantao Zheng, Rui Zhang, Yongshun Li, Ailing Luo, Nan Zhang, Guizhi Shi, Yanfang Wang, Hao Ying, Zhonghua Liu, Hongmei Wang, Anming Meng, Qi Zhou, Hong Wei, Feng Liu, Jianguo Zhao, "Thyroid hormone regulates hematopoiesis via the TR-KLF9 axis", Blood, 2017, 130 (20), pages 2161-2170, [online], [retrieved on 2023-03-13] Retrieved from <URL: https://doi.org/10.1182/blood-2017-05-783043>
Non-Patent Literature 2: Wilkinson, A.C., Ishida, R., Kikuchi, M. et al. "Long-term ex vivo hematopoietic-stem-cell expansion allows nonconditioned transplantation.", Nature, 2019, 571, pages 117-121, [online], [retrieved on 2023-03-24] Retrieved from <URL: https://doi.org/10.1038/s41586-019-1244-x>
Non-Patent Literature 3: Fares, Iman, Chagraoui Jalila, Gareau Yves, Gingras Stephane, Ruel Rejean, Mayotte Nadine, Csaszar Elizabeth, Knapp David J. H. F., Miller Paul, Ngom Mor, Imren Suzan, Roy Denis-Claude, Watts Kori L., Kiem Hans-Peter, Herrington Robert, Iscove Norman N., Humphries R. Keith, Eaves Connie J., Cohen Sandra, Marinier Anne, Zandstra Peter W., Sauvageau Guy, "Pyrimidoindole derivatives are agonists of human hematopoietic stem cell self-renewal", Science, 2014-09-19, 345(6203), pages 1509-1512, [online], [retrieved on 2023-03-20] Retrieved from <URL: https://doi.org/10.1126/science.1256337>
Non-Patent Literature 4: Sakurai, M., Ishitsuka, K., Ito, R. et al. Chemically defined cytokine-free expansion of human hematopoietic stem cells. Nature, 2023, 615, pages 127-133, [online], [retrieved on 2023-04-19] Retrieved from <URL: https://doi.org/10.1038/s41586-023-05739-9>
Non-Patent Literature 5: Kobayashi Hiroshi, Morikawa Takayuki, Okinaga Ayumi, Hamano Fumie, Hashidate-Yoshida Tomomi, Watanuki Shintaro, Hishikawa Daisuke, Shindou Hideo, Arai Fumio, Kabe Yasuaki, Suematsu Makoto, Shimizu Takao; Takubo, Keiyo, "Environmental Optimization Enables Maintenance of Quiescent Hematopoietic Stem Cells Ex Vivo", Cell Reports, 2019-07-02, 28 (1), pages 145-158, [online], [retrieved on 2023-03-13] Retrieved from <URL: https://doi.org/10.1016/j.celrep.2019.06.008>
Non-Patent Literature 6: Bolger Michael and Jorgensen Eugene, "Molecular interactions between thyroid hormone analogs and the rat liver nuclear receptor, Partitioning of equilibrium binding free energy changes into substituent group interactions" The Journal of biological chemistry, 255, 21, 10271-8, [online], [retrieved on 2023-03-27] Retrieved from <URL: https://doi.org/10.1016/S0021-9258(19)70460-8>
Non-Patent Literature 7: Grazia Chiellini, James W. Apriletti, Hikari Al Yoshihara, John D. Baxter, Ralff C.J. Ribeiro, Thomas S. Scanlan, "A high-affinity subtype-selective agonist ligand for the thyroid hormone receptor, Chemistry & Biology", Volume 5, Issue 6, 1998, Pages 299-306, [online], [retrieved on 2023-03-27] Retrieved from <URL: https://doi.org/10.1016/S1074-5521(98)90168-5>
Non-Patent Literature 8: Lorenzini L, Nguyen NM, Sacripanti G, Serni E, Borso M, Saponaro F, Cecchi E, Simoncini T, Ghelardoni S, Zucchi R and Saba A (2019) Assay of Endogenous 3,5-diiodo-L-thyronine (3,5-T2) and 3,3' -diiodo-L-thyronine (3,3' -T2) in Human Serum: A Feasibility Study. Front. Endocrinol. 10:88. [online], [retrieved on 2023-06-08] Retrieved from <URL: https://doi.org/10.3389/fendo.2019.00088>

### SUMMARY OF THE INVENTION

### TECHNICAL PROBLEM

The present invention aims to provide a suitable means for expanding hematopoietic stem cells and other hematopoietic cells capable of cell division while suppressing their differentiation.

### SOLUTION TO PROBLEM

<1> A culture medium for expanding hematopoietic cells capable of cell division, comprising a compound (I) of the following formula or a pharmaceutically acceptable salt thereof: 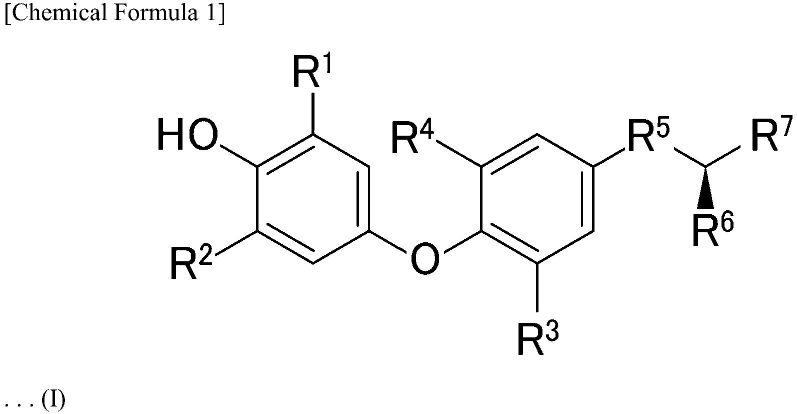 In the formula,
   R¹, R², and R³ are each hydrogen, straight-chain or branched C₁-C₄ alkyl, bromo, or iodo; in one embodiment, R¹, R², and R³ are not all hydrogen;
   R⁴ is straight-chain or branched C₁-C₄ alkyl, bromo, or iodo,
   R⁵ is oxygen, methylene, or a direct bond,
   R⁶ is hydrogen or -NH₂,
   R⁷ is hydrogen or -COOH, provided that R⁶ and R⁷ are not both hydrogen.
<2> The above medium wherein
   (1) R⁵ is oxygen or methylene,
      R⁶ is hydrogen or -NH₂,
      R⁷ is hydrogen or -COOH, provided that R⁶ and R⁷ are not both hydrogen,
         or
   (2) R⁵ is a direct bond,
      R⁶ is hydrogen,
      R⁷ is -COOH.
<3> The above medium wherein,
   Compound (I) is any one of: 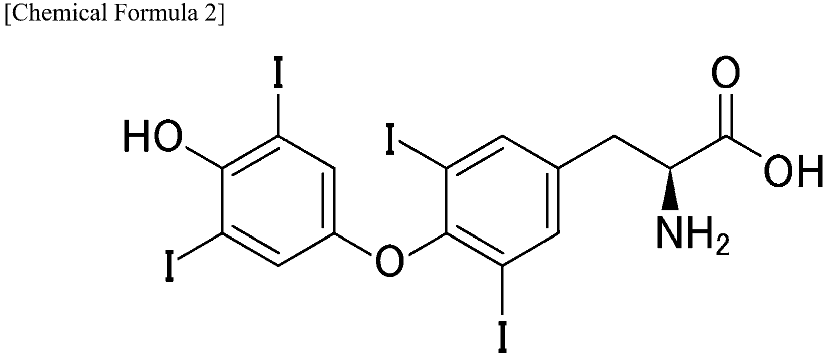
   thyroxine (T₄), 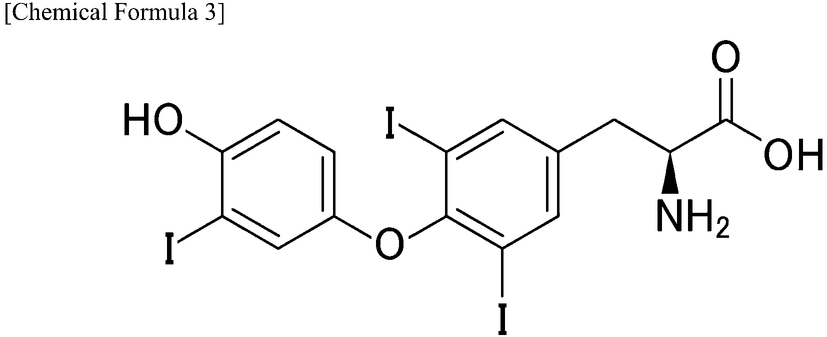
   3,3',5-triiodo-L-thyronine (T₃), 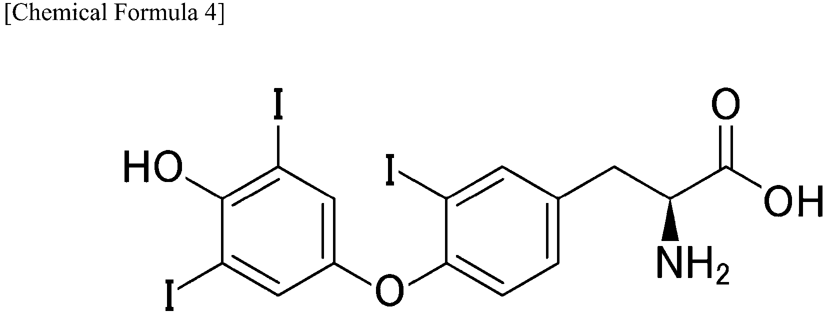
   3,3',5'-Triiodothyronine (rT₃), 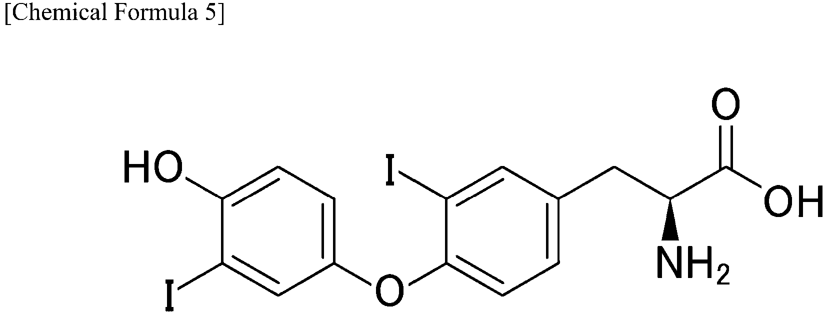
   3,3'-Diiodo-L-thyronine (3,3'-T₂), 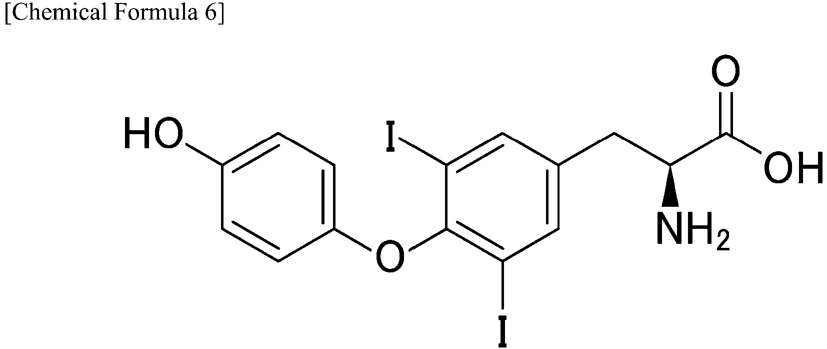
   3,5-Diiodo-L-thyronine (3,5-T₂), 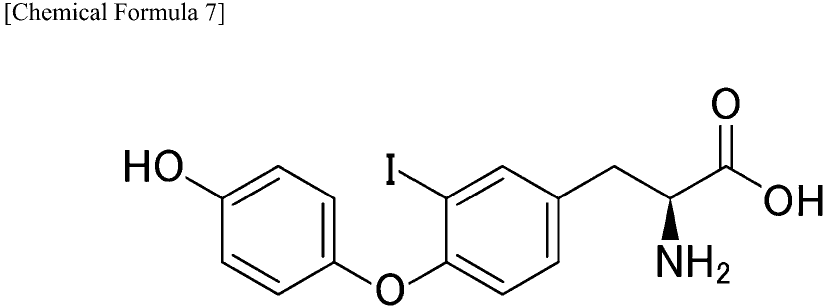
   3-Iodo-L-thyronine (3-T₁), 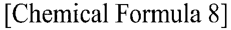 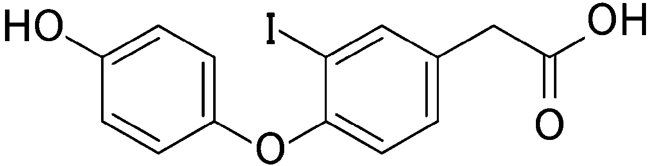
   3-Iodo-L-thyroacetic acid (3-T₁AA), 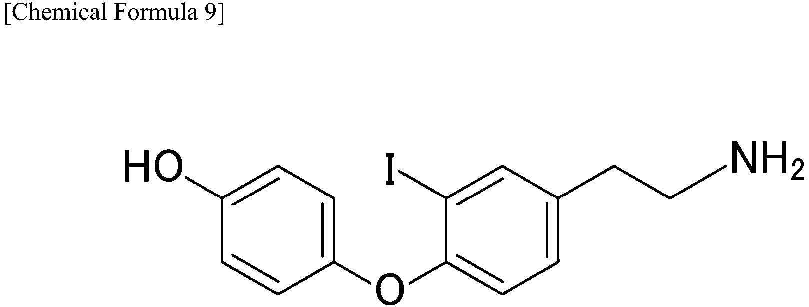
   3-Iodo-L-thyronamine (3-T₁AM), 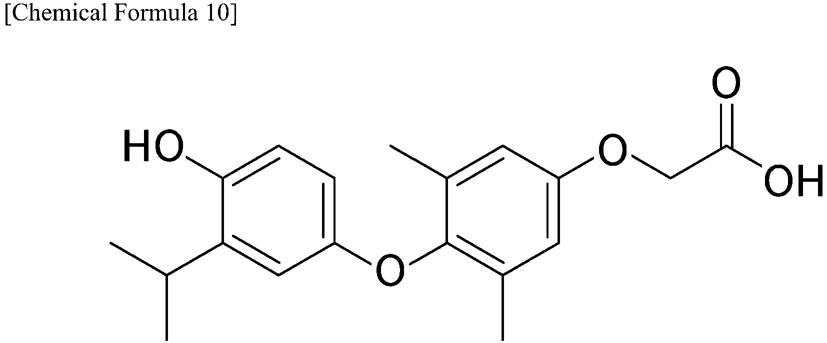
   Sobetirome, 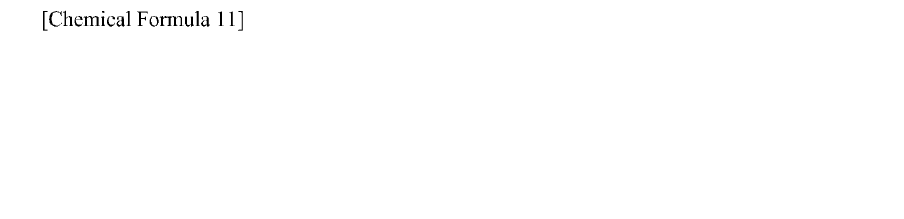 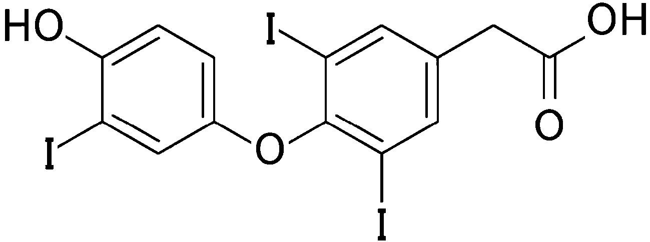
   3,3',5-Triiodo-L-thyroacetic acid (3,3',5-T₃AA) and 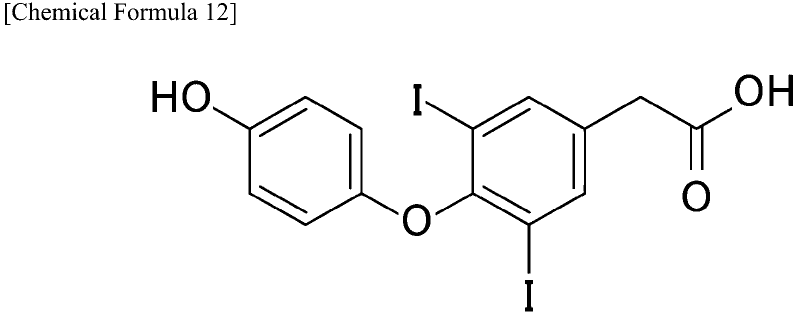
   3,5-Diiodo-L-thyroacetic acid (3,5-T₂AA),
   preferably any one of thyroxine (T₄), 3,3',5-Triiodo-L-thyronine (T₃), 3,3',5'-Triiodothyronine (rT₃), 3,3'-Diiodo-L-thyronine (3,3'-T₂), 3,5-diiodo-L-thyronine (3,5-T₂), and sobetirome, 3,3',5-triiodo-L-thyroacetic acid (3,3',5-T₃AA), and 3,5-diiodo-L-thyroacetic acid (3,5-T₂AA).
<4> The above medium wherein
   R¹ is hydrogen,
   R² and R³ are such that one is hydrogen, straight-chain or branched C₁-C₄ alkyl, bromo, or iodo, and the other is hydrogen.
<5> The above medium wherein compound (I) is either 3,5-diiodo-L-thyronine (3,5-T₂) or 3,3'-diiodo-L-thyronine (3,3'-T₂).
<6> The above medium wherein
   the hematopoietic cells are obtained from an aged donor, or
   the hematopoietic cells are obtained from umbilical cord blood.
<7> The above medium for expanding the hematopoietic cells without mixing them with hematopoietic cells at other differentiation stages.
<8> The above medium for expanding the hematopoietic cells and further expanding differentiated hematopoietic cells generated during the process of expanding the hematopoietic cells.
<9> The above medium wherein
   the aforementioned hematopoietic cells are any one of:
   hematopoietic stem cells that have not yet differentiated into multipotent progenitor cells, multipotent progenitor cells that have completed differentiation from hematopoietic stem cells but have not yet differentiated into precursor cells of differentiated blood cells, and differentiated blood cells that have completed differentiation from their precursor cells
<10> The above medium wherein
   the medium is serum-free and comprises compound (I), wherein compound (I) is, for example, 1 fM to 1 mM, preferably 1 pM to 1 mM, preferably 1 nM to 1 mM, or preferably 0.1 µM to 1 mM, or the medium is a serum medium and comprises compound (I) at 0.1 µM to 1 mM.
<11> A method for producing a clone of hematopoietic cells comprising expanding hematopoietic cells capable of cell division in vitro while applying compound (I) of the following formula or a pharmaceutically acceptable salt thereof to said hematopoietic cells: 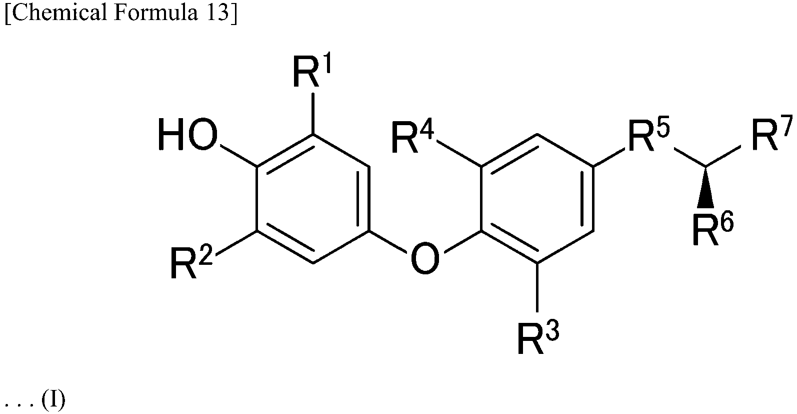 wherein
   R¹, R², and R³ are each hydrogen, straight-chain or branched C₁-C₄ alkyl, bromo, or iodo; in one embodiment, none of R¹, R², and R³ are not all hydrogen;
   R⁴ is straight-chain or branched C₁-C₄ alkyl, bromo, or iodo,
   R⁵ is oxygen, methylene, or a direct bond,
   R⁶ is hydrogen or -NH₂,
   R⁷ is hydrogen or -COOH, provided that R⁶ and R⁷ are not both hydrogen.
<12> A clone of hematopoietic cells obtained by applying compound (I) of the formula below or a pharmaceutically acceptable salt thereof when expanding hematopoietic cells capable of cell division in vitro, wherein: 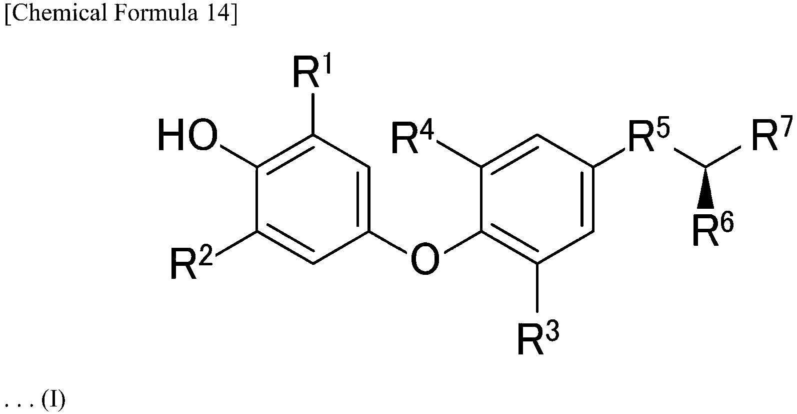 wherein
   R¹, R², and R³ are each hydrogen, straight-chain or branched C₁-C₄ alkyl, bromo, or iodo, and in one embodiment, R¹, R², and R³ are not all hydrogen,
   R⁴ is straight-chain or branched C₁-C₄ alkyl, bromo, or iodo,
   R⁵ is oxygen, methylene, or a direct bond,
   R⁶ is hydrogen or -NH₂,
   R⁷ is hydrogen or -COOH, provided that R⁶ and R⁷ are not both hydrogen,
   the seeded hematopoietic cells are obtained from an aged donor.
<13> An agent for adding to serum medium or serum-free medium to expand hematopoietic cells capable of cell division in vitro,
   comprising as an active ingredient a compound (I) of the following formula or a pharmaceutically acceptable salt thereof: 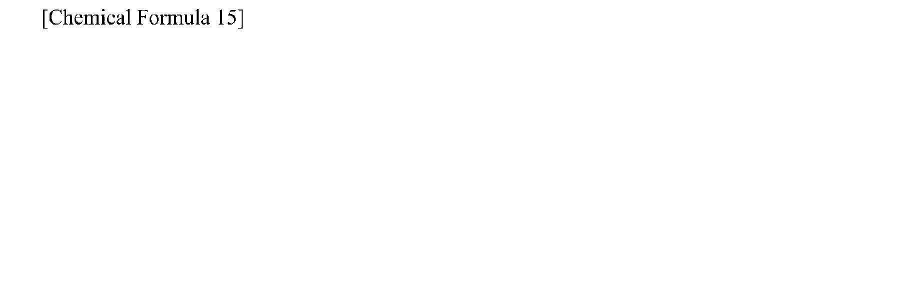 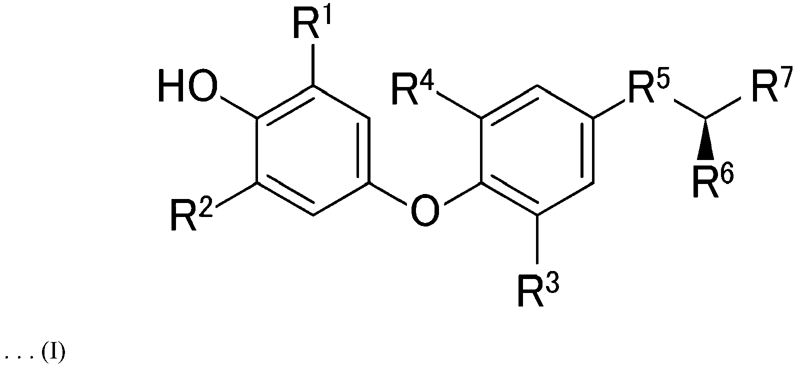
   wherein
      R¹, R², and R³ are each hydrogen, straight-chain or branched C₁-C₄ alkyl, bromo, or iodo;
      R⁴ is straight-chain or branched C₁-C₄ alkyl, bromo, or iodo,
      R⁵ is oxygen, methylene, or a direct bond,
      R⁶ is hydrogen or -NH₂,
      R⁷ is hydrogen or -COOH, provided that R⁶ and R⁷ are not both hydrogen.

### EFFECT OF INVENTION

The present invention provides a suitable means for expanding hematopoietic stem cells and other hematopoietic cells capable of cell division while suppressing their differentiation.

### BRIEF DESCRIPTION OF DRAWINGS

Figure 1 is a graph showing the total cell count in each colony.
Figure 2 is a graph showing the mean fluorescence intensity (MFI) of CD48 staining in each colony.
Figure 3 is a graph showing the change in cell number during culture.
Figure 4 is a figure showing an overview of the chimerism experiment.
Figure 5 is a graph showing the results of the chimerism analysis.
Figure 6 is a graph showing the number of hematopoietic stem cells in each colony.
Figure 7 is a graph showing the results of the chimerism analysis.
Figure 8 is a graph showing the percentage of each type of blood cell in the mouse.
Figure 9 is a graph showing the percentage of EPCR-positive HSCs (EPCR+HSCs) after culturing HSCs in the presence of a thyronine carboxylic acid derivative.
Figure 10 is a graph showing the cell counts after culturing various hematopoietic cells for 4 weeks in the presence of T2.

### DESCRIPTION OF EMBODIMENTS

### <Culture Medium Containing Compound>

A mode of the present embodiment is a culture medium for expanding hematopoietic stem cells and other hematopoietic cells capable of cell division. The medium is, for example, a liquid medium. The medium contains compound (I) of the formula below or a pharmaceutically acceptable salt thereof. 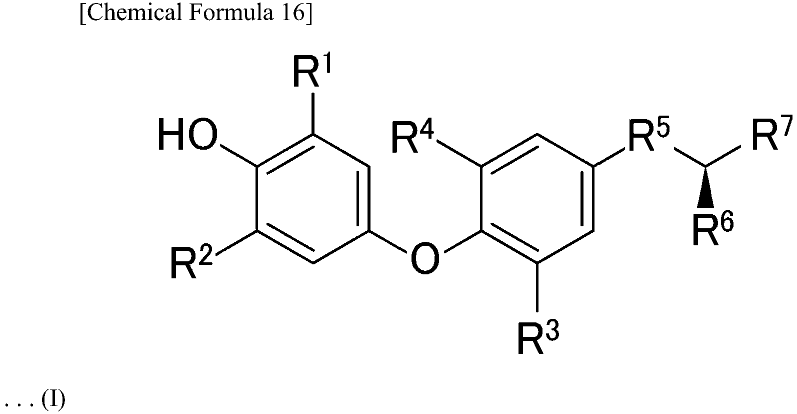
R¹, R², and R³ are each hydrogen, straight-chain or branched C₁-C₄ alkyl, bromo, or iodo. For example, they are methyl, ethyl, isopropyl, or iodo. In one aspect, R¹, R², and R³ are not all hydrogen.
R⁴ is straight-chain or branched C₁-C₄ alkyl, bromo, or iodo. For example, it is methyl, ethyl, isopropyl, or iodo.
R⁵ is oxygen, methylene, or a direct bond; R⁶ is hydrogen or -NH₂; and R⁷ is hydrogen or - COOH. However, R⁶ and R⁷ are not both hydrogen. For example, R⁵ is oxygen, R⁶ is hydrogen, and R⁷ is -COOH. For example, R⁵ is methylene, R⁶ is -NH₂, and R⁷ is -COOH.

In one aspect, R⁵ is oxygen or methylene, R⁶ is hydrogen or -NH₂, and R⁷ is hydrogen or -COOH. However, R⁶ and R⁷ are not both hydrogen. In another aspect, R⁵ is a direct bond, R⁶ is hydrogen, and R⁷ is -COOH.

Preferably, R¹ is hydrogen, and
one of R² and R³ is hydrogen, straight-chain or branched C₁-C₄ alkyl, bromo, or iodo. For example, it is methyl, ethyl, isopropyl, or iodo, and the other is hydrogen.
R⁴ is straight-chain or branched C₁-C₄ alkyl, bromo, or iodo, for example methyl, ethyl, isopropyl, and iodo,
R⁵ is oxygen, methylene, or a direct bond, R⁶ is hydrogen or -NH₂, and R⁷ is hydrogen or - COOH. However, R⁶ and R⁷ are not both hydrogen. For example, R⁵ is oxygen, R⁶ is hydrogen, and R⁷ is -COOH. For example, R⁵ is methylene, R⁶ is -NH₂, and R⁷ is -COOH.

In another aspect of the present embodiment, compound (I) is a thyroid hormone or a mimetic thereof. Compound (I) is, for example, any of the following: 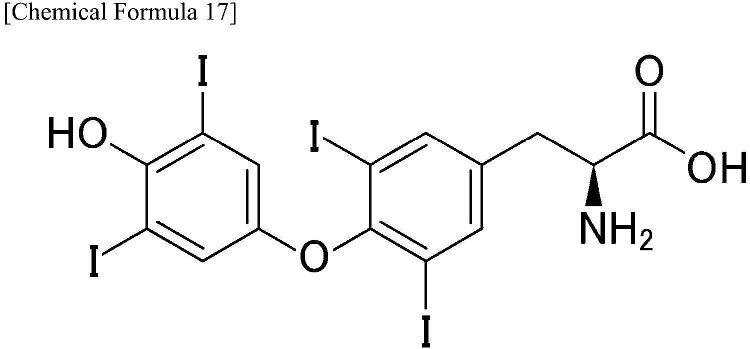
thyroxine (T₄), 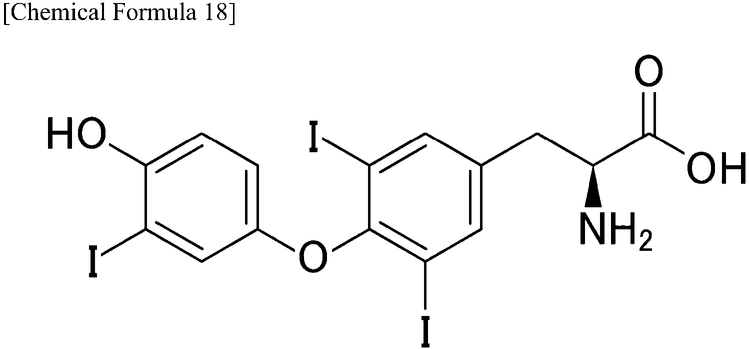
3,3',5-triiodo-L-thyronine (T₃), 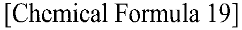 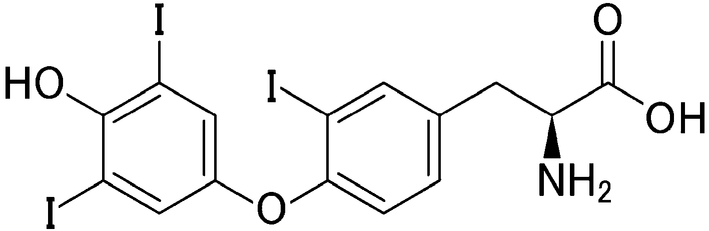
3,3',5'-Triiodothyronine (rT₃), 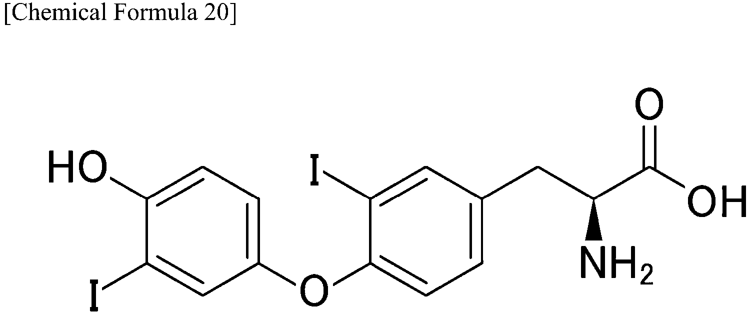
3,3'-Diiodo-L-thyronine (3,3'-T₂), 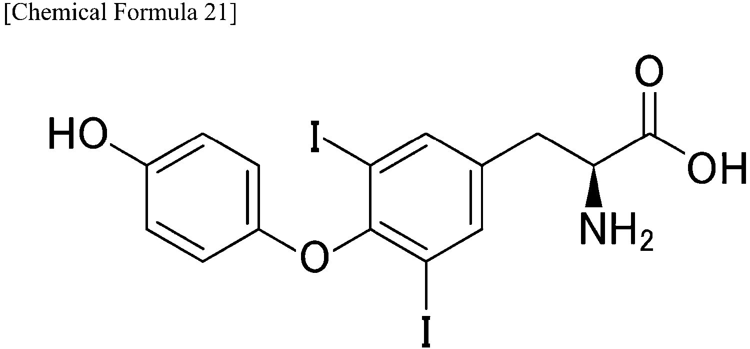
3,5-Diiodo-L-thyronine (3,5-T₂), 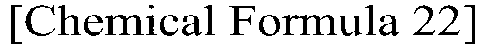 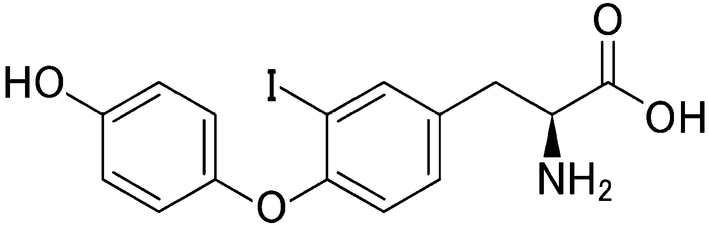
3-Iodo-L-thyronine (3-T₁), 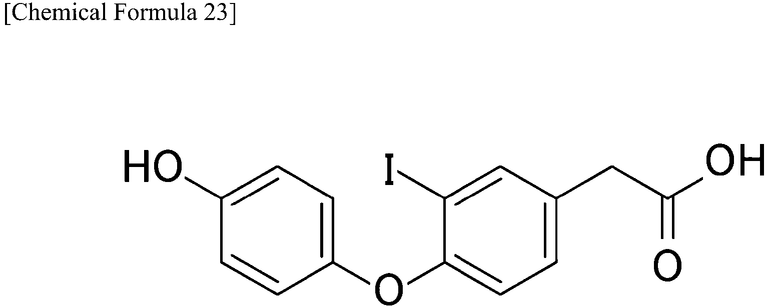
3-Iodo-L-thyroacetic acid (3-T₁AA), 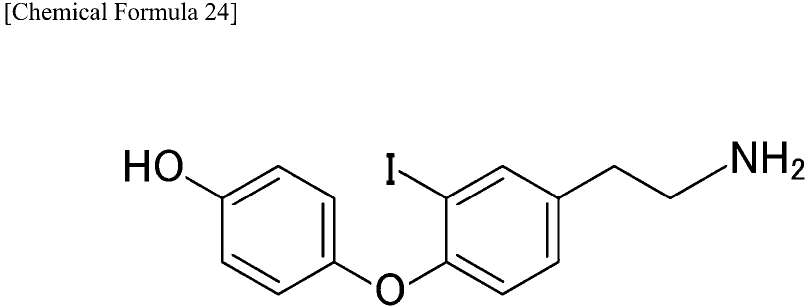
3-Iodo-L-thyronamine (3-T₁AM), 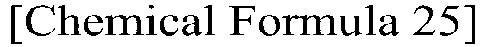 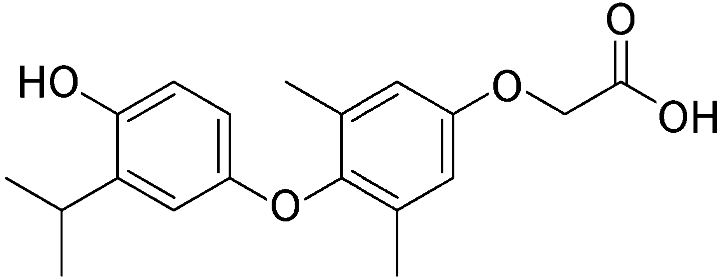
Sobetirome, 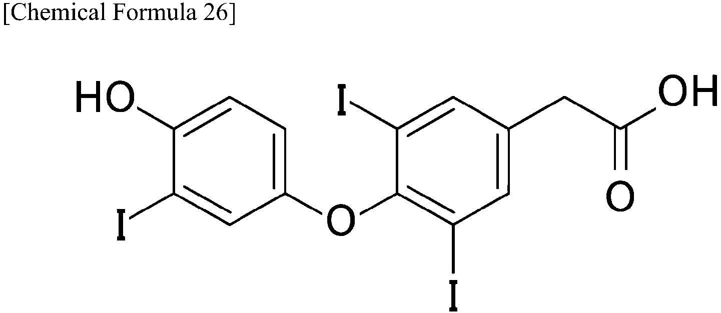
3,3',5-Triiodo-L-thyroacetic acid (3,3',5-T₃AA) and 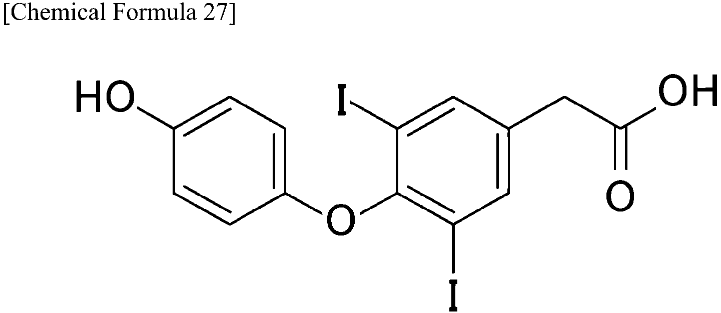
3,5-Diiodo-L-thyroacetic acid (3,5-T₂AA),

Compound (I) is preferably any one of thyroxine (T₄), 3,3',5-Triiodo-L-thyronine (T₃), 3,3',5'-Triiodothyronine (rT₃), 3,3'-Diiodo-L-thyronine (3,3'-T₂), 3,5-diiodo-L-thyronine (3,5-T₂), and sobetirome.

Culturing hematopoietic stem cells in a medium containing compound (I) allows expansion of these cells while suppressing their differentiation, particularly differentiation into multipotent progenitor cells (hematopoietic progenitor cells) and subsequent lineage-specific differentiation. Expansion results in the generation of a large number of clones from the seeded hematopoietic stem cells. From this perspective, preferred compounds (I) are either 3,5-diiodo-L-thyronine (3,5-T₂) or 3,3'-diiodo-L-thyronine (3,3'-T₂). These diiodo-L-thyronines (T₂) promote expansion of hematopoietic stem cells like other compounds, yet exhibit particularly high efficacy in suppressing their differentiation. Similar effects are obtained for other hematopoietic cells capable of cell division.

In preparing compound (I), free thyroid hormone may be extracted from blood. Compound (I) can be chemically synthesized by known methods, such as those described in Non-Patent Literature 6. For example, the synthesis of sobetirome may be performed as described in Non-Patent Literature 7.

In one embodiment, the concentration of compound (I) in the medium is from 1 fM to 1 mM, preferably from 1 pM to 1 mM, more preferably from 1 nM to 1 mM, and most preferably from 0.1 µM to 1 mM. The concentration of compound (I) in the medium may be any of 1, 2, 5, 10, 20, 50, and 100 µM.

### <Agent Applied to Hematopoietic Cells>

Another aspect of the present embodiment is an agent applied to hematopoietic cells capable of cell division to expand them in vitro. The agent contains compound (I) of the above formula or a pharmaceutically acceptable salt thereof as an active ingredient. The agent may be a medium additive. The agent may be added to serum-containing medium or serum-free medium. The agent may be applied to cells prior to expanding them in the medium.

### <Base Medium>

The composition of the base medium to which compound (I) should be added is not particularly limited, provided it readily suppresses the differentiation of hematopoietic stem cells and other hematopoietic cells capable of cell division, or in other words, readily maintains their undifferentiated state. In one embodiment, the base medium is serum-free medium. The medium to which compound (I) is added is also serum-free medium. In another embodiment, the base medium is serum medium.

The base medium to which compound (I) is added, particularly the serum medium, contains compound (I) at a concentration of 0.1 µM to 1 mM. This concentration range is significantly higher than, for example, the blood concentration of free thyroxine (T₄) in humans, which is typically 0.8 to 1.8 (ng/dl). Furthermore, according to Non-Patent Literature 8, the blood concentration of free 3,5-diiodo-L-thyronine (3,5-T₂) in humans is 78 ± 5 pM, and the blood concentration of free 3,3'-diiodo-L-thyronine (3,3'-T₂) in humans is 253 ± 29 pM. The concentration range of these compounds (I) added as active ingredients to serum culture medium is sufficiently higher than these values.

The base medium may be any medium typically used for cell culture, or a medium prepared for hematopoietic stem cell culture, without particular limitation. For example, media commonly used for cell culture include αMEM, DMEM, Ham's F12 medium, DMEM/F12 medium, RPMI 1640 medium, IMEM, McCoy's 5A medium, and EMEM. Media used for hematopoietic stem cell culture include S-Clone media (e.g., SF-O3 medium, EDIALAB), StemPro 34 (Invitrogen), Stemline (Sigma-Aldrich), Stemline II (Sigma-Aldrich), X-VIVO 10 (Lonza), X-VIVO 15 (Lonza), X-VIVO 20 (Lonza), HPGM (Lonza), StemSpan H3000 (StemCell Technologies), StemSpan SFEM (Serum-Free Expansion Medium, StemCell Technologies), QBSF-60 (Quality Biologicals). In this embodiment, these media may be appropriately mixed and used. These basal media can also be favorably used for other hematopoietic cells capable of cell division.

The base medium according to this embodiment is not limited to the above examples. However, from the perspective of having a track record of use in hematopoietic stem cell culture, it is preferably SF-O3 medium, αMEM, or DMEM/F12 medium, and more preferably SF-O3 medium. As shown in the examples described later, StemSpan Serum-Free Expansion Medium is also suitable for use with human hematopoietic stem cells. These base mediuma are also suitable for use with other hematopoietic cells capable of cell division.

### <Other Medium Additives>

In one embodiment, "stem cell factor (SCF)" is added to the base medium. SCF is a type of hematopoietic factor (cytokine). Specifically, it is a ligand for the tyrosine kinase receptor encoded by the KIT gene locus. SCF is also referred to as KIT ligand or KITLG. Human-derived SCF is a protein comprising the amino acid sequence described in NCBI Reference Sequence: NP_000890 or NP_003985. Mouse-derived SCF is a protein comprising the amino acid sequence described in NCBI Reference Sequence: NP_001334085 or NP_038626.

In one embodiment, the concentration of SCF is from 10 to 1,000 ng/mL. The concentration may be 20, 50, 100, 200, or 500 ng/mL.

In one embodiment, "thrombopoietin (TPO)" is added to the base medium. TPO is a type of hematopoietic factor (cytokine). Specifically, it is a glycoprotein involved in the proliferation and differentiation of platelet precursor cells (megakaryocytes). TPO is also referred to as THPO, thrombopoietic stimulator (TSF), megakaryocyte colony-stimulating factor (MK-CSF), megakaryocyte stimulator, or megakaryocyte proliferation factor. Human TPO is a protein consisting of the amino acid sequences described in NCBI Reference Sequences: NP_000451, NP_001171068, NP_001171069, NP_001276926, or NP_001276927. Mouse-derived TPO is a protein consisting of the amino acid sequence described in NCBI Reference Sequence: NP_001166976, NP_001276823, NP_001276825, or NP_033405.

In one embodiment, the concentration of TPO is from 10 to 1,000 ng/mL. The concentration may be 20, 50, 100, 200, or 500 ng/mL.

In one embodiment, "low-density lipoprotein (LDL)" is added to the base medium. In one embodiment, the LDL concentration is from 10 to 1,000 ng/mL. The concentration may be any of 20, 50, 100, 200, and 500 ng/mL.

In one embodiment, "Flt3 ligand (Fms-like tyrosine kinase 3 ligand)" is added to the base medium. In one embodiment, the concentration of Flt3 ligand is from 10 to 1,000 ng/mL. The concentration may be any of 20, 50, 100, 200, and 500 ng/mL.

In one embodiment, "low-density lipoprotein (LDL)" is added to the base medium. In one embodiment, the concentration of LDL is from 10 to 1,000 ng/mL. The concentration may be any of 20, 50, 100, 200, and 500 ng/mL.

In one embodiment, a compound selected from the group consisting of UM171, Soluplus, 740Y-P, and butyzamide is added to or not added to the base medium. When UM171 is added, its concentration is, for example, 3.5 to 350 nM. The concentration may be 7, 17.5, 35, 70, or 175 nM. 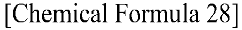 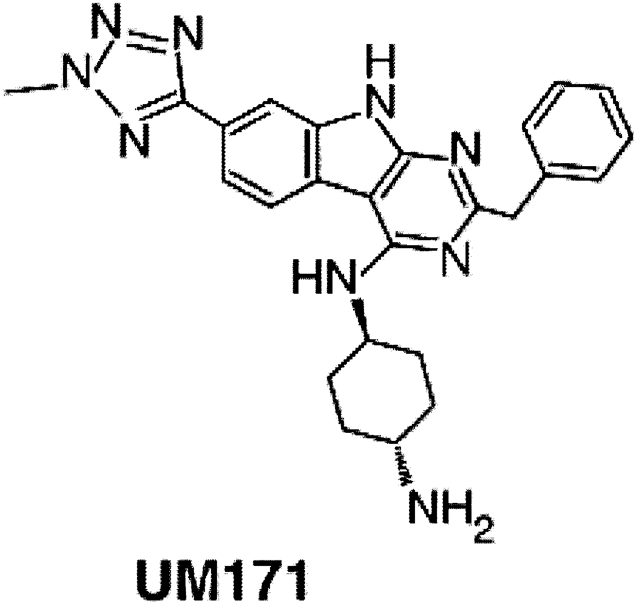 Quoted from Non-Patent Literature 3

In one embodiment, polyvinyl alcohol (PVA) is added to or not added to the base medium. In one embodiment, the base medium does not contain PVA, UM171, Soluplus, 740Y-P, butyzamide, or other components not derived from living organisms.

Furthermore, the medium of this embodiment may additionally contain conventional medium additives known in the art. Such medium additives include, for example, functional proteins (insulin, transferrin, lactoferrin, etc.), lipids other than fatty acids (cholesterol, etc.), reducing agents (2-mercaptoethanol, catalase, superoxide dismutase, N-acetylcysteine, etc.), amino acids (alanine, L-glutamine, non-essential amino acids, etc.), peptides (glutathione, reduced glutathione, etc.), nucleotides (nucleosides, cytidine, adenosine 5'-monophosphate, hypoxanthine, thymidine, etc.), metal salts (iron(III) nitrate, iron(II) sulfate, copper sulfate, zinc sulfate, etc.), inorganic salts (sodium, potassium, calcium, magnesium, phosphorus, chlorine, etc.), carbon sources (glucose, galactose, fructose, sucrose, etc.), vitamins, inorganic compounds (selenous acid), organic compounds (para-aminobenzoic acid, ethanolamine, corticosterone, progesterone, lipoic acid, putrescine, pyruvic acid, lactic acid, triiodothyronine, etc.), antibiotics (penicillin, streptomycin, etc.), buffer compounds (HEPES, sodium bicarbonate, etc.), pH indicators (phenol red, etc.), but are not limited to these.

A mode of embodiment of the present invention is a culture medium kit that provides two or more of the above compounds (I), base medium, and medium additives in a separated state. By using the culture medium kit, fresh culture medium can be prepared in a timely manner.

### <Culture Environment>

It is preferable to culture hematopoietic stem cells under an oxygen concentration lower than the normal atmospheric oxygen concentration, for example, 20 vol%. Under such hypoxic conditions, the oxygen concentration in the gas in contact with the culture medium during cultivation is preferably 1 to 15 vol%, more preferably 1 to 10 vol%, and most preferably 1 to 5 vol%. The carbon dioxide concentration in the gas is typically 1 to 10 vol%, and preferably 2 to 5 vol%. Culture under such hypoxic conditions can be performed, for example, by culturing within a commercially available hypoxic incubator. The culture temperature is not particularly limited but is typically 30 to 40°C, preferably approximately 37°C. These culture conditions can also be advantageously used for other hematopoietic cells capable of cell division.

Culturewares commonly known for hematopoietic stem cell culture may be suitably utilized. The culturewares may be non-adherent. The culturewares may also be coated with a cell-adherent material, such as a cell support substrate like ECM, Matrigel, gelatin, collagen, etc. Furthermore, hematopoietic stem cell culture may be either non-adherent or adherent. Examples of non-adherent culture include dispersed culture, aggregated suspension culture, and suspension culture on a carrier. These culture vessels and culture formats can also be suitably used for other hematopoietic cells capable of cell division.

The number of cells seeded per unit volume of medium should preferably be selected at a value suitable for cell expansion. For example, 3x10⁰ to 3x10⁶ cells per 1 mL of medium. The cell number may also be 3x10¹, 1x10², 3x10², 1x10³, 3x10³, 1x10⁴, 3x10⁴, 1x10⁵, 3x10⁵, or 1x10⁶ cells per 1 mL of medium.

During medium changes, cells may be partially removed, leaving 1/2 to 1/4 of the cells, such that the number of cells in the medium is 3x10⁰ to 3x10⁶ cells per mL of medium. During medium changes, differentiated hematopoietic stem cells in the medium may be removed once every 1 to 10 medium changes, preferably once every 3 to 4 medium changes. At this time, the hematopoietic stem cells may be purified by processing the cells with a cell sorter. These culture formats may also be suitably used for other hematopoietic cells capable of cell division.

In one embodiment, hematopoietic cells are expanded without mixing them with hematopoietic cells at other differentiation stages. In one embodiment, hematopoietic cells are expanded, and differentiated hematopoietic cells generated during the expansion process of the hematopoietic cells are also expanded. In one embodiment, the hematopoietic cells are hematopoietic stem cells that have not yet differentiated into multipotent progenitor cells. In other embodiments, the hematopoietic cells are multipotent progenitor cells that have already completed differentiation from hematopoietic stem cells but have not yet differentiated into precursor cells of differentiated blood cells. In one embodiment, the hematopoietic cells are differentiated blood cells that have already completed differentiation from their precursor cells. In one embodiment, differentiated blood cells are, for example, monocytes, macrophages, lymphocytes, neutrophils, basophils, and eosinophils. Lymphocytes are, for example, T lymphocytes and B lymphocytes.

In one embodiment, hematopoietic stem cells are cultured without mixing them with other cells. The hematopoietic stem cells are expanded in a state not mixed with other cells, such as bone marrow stromal cells. In another embodiment, hematopoietic stem cells are expanded in a state mixed with other cells, such as bone marrow stromal cells. These culture formats can also be advantageously used for other hematopoietic cells capable of cell division.

In one embodiment, the culture period lasts from 1 day to 6 months. For example, it may be 1, 2, 4, 6, or 13 weeks. In one embodiment, the passage interval is from 5 days to 30 days. For example, it is one of 10, 11, 12, 13, 14, 15, 16, 17, 18, 19, 20, 21, 22, 23, 24, and 25 days. Passaging may be omitted. In one embodiment, the interval between medium changes is 2 to 10 days. For example, it is one of 4, 5, 6, and 7 days. Medium changes may be omitted.

In one embodiment, a culture kit is provided comprising a medium containing compound (I) or a medium kit containing compound (I). The culture kit further comprises, in addition to the medium kit, a culture vessel, a hypoxic incubator, and other cells. In one embodiment, the culture kit further comprises reagents for confirming that hematopoietic stem cells maintain their undifferentiated state. Such reagents include antibodies recognizing markers described below, labeled secondary antibodies recognizing said antibodies, and magnetic beads conjugated to either of these antibodies. Furthermore, the kit may include instructions detailing conditions for expanding hematopoietic stem cells. These kit formats are also suitable for use with other hematopoietic cells capable of cell division.

### <Preparation of Hematopoietic Stem Cells and Other Mitotic Blood Lineage Cells to be Expanded>

From one perspective, "hematopoietic stem cells" are cells possessing multipotency, capable of differentiating into all blood cell lineages of the hematopoietic system, and capable of self-renewal while maintaining this multipotency. From one perspective, "hematopoietic cells" are cells of cell lineages differentiable from hematopoietic stem cells. From one perspective, hematopoietic cells capable of cell division are hematopoietic cells excluding erythrocytes and platelets. Multipotent progenitor cells differentiate from hematopoietic stem cells. Precursor cells for each differentiated blood cell, such as granulocyte precursor cells, differentiate from multipotent progenitor cells. Each differentiated blood cell, such as leukocytes, differentiates from such precursor cells.

The animal species from which the hematopoietic stem cells originate, i.e., the donor, is not particularly limited, but vertebrates are preferred, and mammals are more preferred. Examples of mammals include, but are not limited to, primates such as humans, monkeys, and marmosets; rodents such as mice and rats; lagomorphs such as rabbits; ungulates such as pigs, sheep, cattle, goats, and horses; and Carnivora such as dogs and cats. The hematopoietic stem cells according to this embodiment are preferably hematopoietic stem cells derived from rodents such as mice or primates such as humans, and more preferably are human-derived hematopoietic stem cells. The same applies to other hematopoietic cells capable of cell division.

The donor's age is not particularly restricted. Applying the method of the present embodiment to hematopoietic stem cells collected from an aged donor, particularly primary cultured hematopoietic stem cells, is expected to functionally rejuvenate these hematopoietic stem cells. Here, an "aged donor" may be an adult. For humans, for example, an adult may be aged 20 to 100 years, or further narrowed to 40 to 100 years. However, if the donor was age of weeks or years capable of pregnancy, the phrase "hematopoietic stem cells collected from the donor" excludes hematopoietic stem cells collected from the fetal or neonatal side relative to the donor's placenta, such as those derived from umbilical cord blood. Since hematopoietic stem cells derived from umbilical cord blood are considered to originate from fetal or neonatal cells rather than maternal cells, they may not be suitable for the rejuvenation expected from the processing method of this embodiment. Furthermore, the range of "aged donors" may be narrowed to include only older individuals. When the donor is a mouse, an aged donor may be an individual between, for example, 80 weeks of age and the end of its natural lifespan. When the donor is a human, the age of the aged donor may be, for example, from 56 to 100 years old. In one embodiment, hematopoietic stem cells derived from an aged donor exhibit reduced differentiation capacity into cells of the T lymphocyte lineage compared to hematopoietic stem cells derived from a younger donor. In one embodiment, rejuvenation of hematopoietic stem cells means an increase in their ability to differentiate into cells of the T lymphocyte lineage. Thus, a method for restoring the function of hematopoietic stem cells that declines with aging was previously unknown. Hematopoietic stem cells collected from an aged donor may be rejuvenated and then autologously transplanted. The same applies to other hematopoietic cells capable of cell division.

Furthermore, while there are no particular restrictions on the tissue from which hematopoietic stem cells are derived as long as the cells are present, it is preferable that the tissue be hematopoietic. Examples include bone marrow, umbilical cord blood, peripheral blood, and liver. The same applies to other hematopoietic cells capable of cell division.

Hematopoietic stem cells may also be induced from pluripotent stem cells such as iPS cells or ES cells. Such pluripotent stem cells may be cells provided with a defect in antigen presentation via HLA class I molecules, so-called universal cells. Hematopoietic stem cells induced from such universal cells are transplanted into the recipient. Hematopoietic stem cells derived from universal cells are less susceptible to immune rejection after transplantation. The same applies to other hematopoietic cells capable of division.

Hematopoietic stem cells are typically isolated from the above tissues using surface markers specifically expressed on these cells (hematopoietic stem cell-specific markers) or surface markers not expressed on these cells as indicators. Regarding the presence or absence of such marker expression, human-derived hematopoietic stem cells are typically CD34-positive (+) cells or CD34-positive (+) CD38-negative (-) CD90 positive (+) CD45RA negative (-) cells, and preferably CD34⁺ CD38⁻ CD90⁺ CD45RA⁻ CD49f⁺ cells. In other embodiments, human-derived hematopoietic stem cells are cells that are CD34⁺ CD38⁻ CD90⁺ CD45RA⁻ EPCR^{+.} Furthermore, mouse-derived hematopoietic stem cells are typically CD150⁺ CD48⁻ Flt3⁻ LSK cells, and preferably CD150⁺ CD48⁻ CD41⁻ CD34⁻ Flt3⁻ LSK cells. In other embodiments, human-derived hematopoietic stem cells are cells that are CD150⁺ CD48⁻ EPCR⁺ LSK. Note that "LSK" refers to Lineage⁻ Sca-1⁺ c-Kit⁺ cells. In one embodiment, the hematopoietic stem cells to be expanded have not yet differentiated into hematopoietic progenitor cells.

When cells are bound together by stroma within the hematopoietic tissue, they are first dissociated into individual cells by treatment with proteases, collagenases, etc. On the other hand, if the cells are already separated, such as in blood tissue like peripheral blood, they are used for isolating the hematopoietic stem cells described below without further cell separation. The same applies to other hematopoietic cells capable of cell division.

There are no particular restrictions on the method for isolating hematopoietic stem cells; it can be performed using appropriate known methods. For example, as shown in the examples described later, cells possessing hematopoietic stem cell-specific marker characteristics can be isolated using antibodies against the aforementioned hematopoietic stem cell-specific markers and devices such as cell sorters or magnetic beads. This isolation step purifies and enriches the hematopoietic stem cells. Such purification and concentration may be performed again during passage of the hematopoietic stem cells. The same applies to other hematopoietic cells capable of cell division.

### <Production of Clones of Hematopoietic Stem Cells and Other Mitotically Active Hematopoietic Cells>

One embodiment of the present invention involves the production of clones of hematopoietic stem cells and other hematopoietic cells capable of cell division in vitro. In this process, the hematopoietic stem cells are expanded while applying the above compound (I) or its pharmaceutically acceptable salt to them. One method of applying compound (I) or its salt is to culture the hematopoietic stem cells in a medium containing compound (I) or its salt. This method of production enables the large-scale supply of clones that maintain undifferentiated properties. The same applies to other hematopoietic cells capable of cell division.

Another aspect of this embodiment is a clone of hematopoietic stem cells obtained by expanding hematopoietic stem cells as described above. In this embodiment, a clone refers to a group of identical cells sharing a common ancestor. The clone may originate from a single progenitor. That is, it may be monoclonal. The clone may originate from a few progenitors. That is, it may be oligoclonal. The clone may originate from many progenitors. That is, it may be polyclonal. These clones maintain their undifferentiated state comparable to or younger than that of their progenitors. The same applies to other hematopoietic cells capable of cell division.

In one embodiment, the ancestral hematopoietic stem cells, i.e., the hematopoietic stem cells prior to application of the above compound (I) or its pharmaceutically acceptable salt, are from an aged donor. In such clones, the differentiation capacity into the T lymphocyte lineage is enhanced compared to the ancestors. The same applies to other hematopoietic cells capable of cell division.

In one embodiment, the clone contains cells differentiated into hematopoietic progenitor cells or cells of subsequent lineages. The proportion of clones maintaining undifferentiated state in one embodiment is from 30% to 100%. For example, it is 50% or more, or 70% or more. The proportion of clones maintaining undifferentiated state can be identified by a cell sorter using antibodies against a marker. In one embodiment, the marker is expressed specifically on hematopoietic stem cells and not on differentiated cells. In another embodiment, the marker is not specifically expressed on hematopoietic stem cells but is expressed on differentiated cells. The same applies to other hematopoietic cells capable of cell division.

Whether a clone functions as a hematopoietic stem cell can be determined by transplanting the clone into immunodeficient mice, such as NOG mice or NOD/SCID mice. The clone cells of interest are transplanted into the mouse, and the migration or engraftment of the transplanted cells into the bone marrow, their multipotency, and their bone marrow reconstitution capacity are detected. Based on these detection results, it can be confirmed that the clone maintains its function as a hematopoietic stem cell. Furthermore, the bone marrow of an immunodeficient mouse where transplantation has been established can be collected and transplanted again into another immunodeficient mouse, i.e., secondary transplantation, to measure the self-renewal capacity and long-term bone marrow reconstitution capacity of the transplanted cells. The same applies to other hematopoietic cells capable of cell division.

### <Transplantation and Medicinal Compositions>

The above clones are useful for cell therapy and regenerative medicine. In one embodiment, the above clones are transplanted into a recipient whose bone marrow hematopoietic function is impaired. In one embodiment, the clones can restore bone marrow hematopoietic function by engrafting into the bone marrow. The clone can be used as a graft for hematopoietic stem cell therapy, replacing grafts used in bone marrow or umbilical cord blood transplants. In one embodiment, it is a pharmaceutical composition containing the clone. The same applies to other hematopoietic cells capable of cell division.

The pharmaceutical composition of the present embodiment can be manufactured as a non-oral formulation, such as an injection, suspension, or infusion solution, by mixing with a pharmaceutically acceptable carrier according to conventional methods. Medically acceptable carriers that may be included in such non-oral formulations include, for example, aqueous solutions for injection such as physiological saline, isotonic solutions containing glucose and other excipients (e.g., D-sorbitol, D-mannitol, sodium chloride). The formulation may also be combined with, for example, buffers (e.g., phosphate buffer, sodium acetate buffer), anesthetics (e.g., benzalkonium chloride, procaine hydrochloride), stabilizers (e.g., serum albumin, polyethylene glycol), preservatives, antioxidants, etc.

Clones are also effective in treating various diseases, including the treatment of leukemias requiring bone marrow transplantation. Therefore, hematopoietic stem cells maintained by the method of this embodiment are effective for diseases involving hematopoietic stem cell depletion and/or reduced hematopoietic function, diseases involving impaired hematopoietic function, immune cell depletion, immune cell proliferation, diseases involving autoimmunity, immune dysfunction, and ischemic diseases. The same applies to other hematopoietic cells capable of cell division.

Specifically, diseases related to hematopoietic function include blood cancers such as acute or chronic leukemia, chronic granulomatous disease, severe combined immunodeficiency syndrome, adenosine deaminase (ADA) deficiency, agammaglobulinemia, Wiskott-Aldrich syndrome, Chediak-Higashi syndrome, acquired immunodeficiency syndrome (AIDS), C3 deficiency, thalassemia, congenital anemias such as hemolytic anemia due to enzyme deficiency and sickle cell anemia, lysosomal storage disorders such as Gaucher disease and mucopolysaccharidoses, adrenoleukodystrophy, Fanconi syndrome, aplastic anemia, granulocytopenia, lymphopenia, thrombocytopenia, idiopathic thrombocytopenic purpura, thrombotic thrombocytopenic purpura, Kasabach-Merritt syndrome, malignant lymphoma, Hodgkin's disease, multiple myeloma, chronic liver disease, autoimmune hemolytic anemia, myelodysplastic syndrome, polycythemia vera, erythrocytosis, essential thrombocythemia, myeloproliferative disorders, and spinal cord injury due to trauma, etc., are examples.

Transplantation may be allogeneic or autologous. As an example of autologous transplantation, when bone marrow suppression occurs as a side effect of chemotherapy, radiation therapy, etc., in patients with solid tumors, bone marrow can be collected prior to treatment. Subsequently, clones produced by the method of this embodiment can be reintroduced into the patient after treatment, enabling early recovery from hematopoietic system impairment. This method allows for the administration of more potent chemotherapy, etc., thereby enhancing the therapeutic effect.

Furthermore, the clones contained in the pharmaceutical composition of this embodiment may be clones of genetically modified cells for performing gene therapy. Genetic modification may also be applied to the clones. The genes to be modified may be selected according to the target disease and are not particularly limited, but examples include genes for hormones, cytokines, receptors, enzymes, polypeptides, etc. Furthermore, genetic modification can be performed by those skilled in the art using appropriate known techniques as deemed suitable, such as gene transfer using viral vectors, genome editing, knockout methods, siRNA methods, shRNA methods, ribozyme methods, or antisense methods. Furthermore, such gene modification-based therapy targeting hematopoietic stem cells can be applied to treat congenital genetic disorders. Examples of such therapies include: introduction of the ADA gene for adenosine deaminase (ADA) deficiency; introduction of the γc chain gene for X-linked severe combined immunodeficiency (X-SCID); introduction of the gp91-phox gene for chronic granulomatous disease; and introduction of the glucocerebrosidase gene for Gaucher disease. The same applies to other hematopoietic cells capable of cell division.

Furthermore, this embodiment provides a method for treating diseases related to hematopoietic function by administering to a patient the clone obtained in this embodiment or a pharmaceutical composition containing said clone as an active ingredient.

As described above, the clones, pharmaceutical compositions, and diseases related to hematopoietic function are as stated. Furthermore, the administration method for these cells, etc., varies depending on the recipient's age, weight, sex, condition, etc., but they can be administered via either an oral route or a non-oral route (e.g., intravenous administration, arterial administration, local administration). The dosage of clones also varies depending on the subject's age, weight, sex, condition, etc., but can be appropriately adjusted by those skilled in the art.

### EXAMPLES

### <1. Content of the Examples>

Hereinafter, hematopoietic stem cells are denoted as HSC. Hematopoietic stem cells in mice and humans are expanded using thyroid hormones (T₄ and T₃) and their metabolites rT₃ and T₂ (3,3'-Diiodo-L-Thyronine and 3,5-Diiodo-L-thyronine). HSCs are expanded through the simple operation of adding thyroid hormones or their metabolites to conventional HSC culture medium. Furthermore, it functionally rejuvenates aged HSCs.

### <2. Comparison with Prior Art>

(1) Prior Art: Although HSC expansion technology is considered useful for gene/cell therapy and regenerative medicine, it has only recently been established. Molecules such as PVA and UM171 described earlier can expand HSCs in either mice or humans, but not both. These molecules do not naturally exist within the body. That is, physiological HSC proliferation factors have not been identified. Furthermore, while HSCs decline in function with aging, no recovery method using external factors exists.
(2) Differences from Prior Art: This embodiment can be applied to both mouse and human HSCs. The proportion of HSCs within the total expanded cell population is maintained at a higher level than with conventional expansion techniques. Furthermore, this example is not limited to mere expansion technology. This example restores the function of aged HSCs. Specifically, this example (i) improves the engraftment rate of HSCs transplanted into the body. Additionally, (ii) a marked improvement in lymphocyte production capacity is observed. That is, functional rejuvenation of HSCs is observed.

Unlike PVA or UM171, thyroid hormones or their metabolites are physiological factors. Therefore, the biological applicability of these compounds or mimetic compounds is high. Furthermore, their use in cell expansion is simple, requiring only their addition to the culture medium, and the expansion process incurs low costs. However, the combination of PVA or UM171 with thyroid hormones or their metabolites is not excluded from one aspect of the present invention.

(3) Application of Examples: Physiological proliferation factors for HSCs have been identified. This enables improvements in techniques for transplanting hematopoietic stem cells obtained from umbilical cord blood. Furthermore, the methods of the examples are expected to lead to improvements and new developments in gene and cell therapy technologies. Additionally, a technology for the functional rejuvenation of aged HSCs using external factors is provided.

### <3-1. Short-Term Culture of HSCs>

Method: A population containing mouse hematopoietic stem cells was processed using a cell sorter to enrich cells with the surface phenotype CD150⁺ CD48⁻ EPCR⁺ Lineage⁻ Sca-1⁺c-Kit⁺. These purified cells are hereinafter referred to as mouse HSCs. 300 mouse HSCs were cultured for one week at 37°C under 5% CO₂ in medium containing SF-O3 (Sanko Junyaku), SCF 100ng/mL, and TPO 100ng/mL. The number of cells in each colony formed after culture was counted. All subsequent culture experiments were performed at 37°C under 5% CO₂. The concentration of each thyroid hormone and its metabolites was set to 10 µM. The control group (Ctl) medium contained no added thyroid hormones or metabolites.

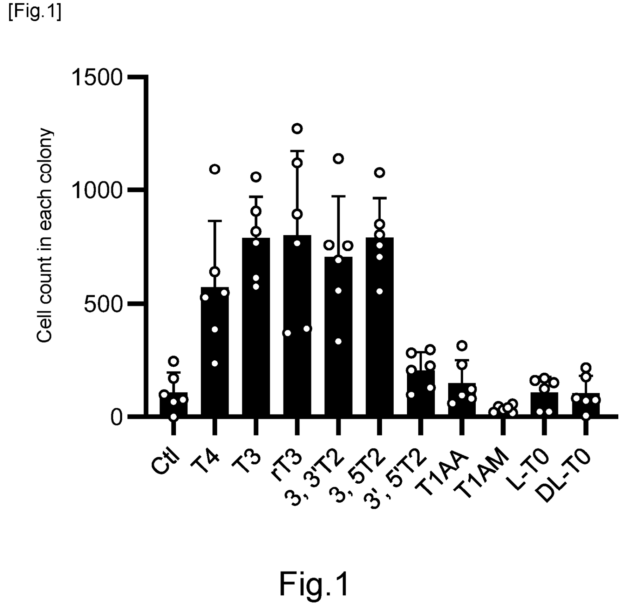

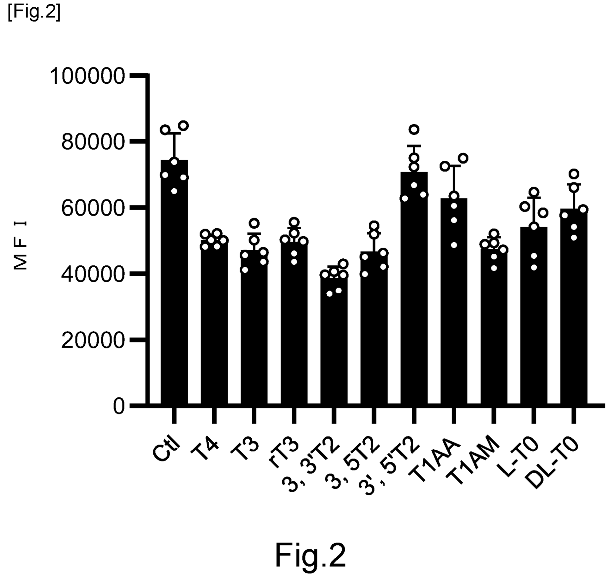

Results: The groups treated with thyroid hormones and their metabolites were compared to the control group (Ctl). The graph in Figure 1 shows the mean total cell count per colony. Circles represent counts per colony. Error bars indicate standard deviation. "Ctl" on the x-axis denotes the absence of added thyroid hormones and their metabolites. The graph in Figure 2 shows the mean fluorescence intensity (MFI) of CD48 staining per colony. Circles represent measured values per colony. Error bars indicate standard deviation.

In Figures 1 and 2, T4 denotes thyroxine (T₄). T3 denotes 3,3',5-triiodo-L-thyronine (T₃). rT3 denotes 3,3',5'-triiodothyronine (rT₃). 3,3'T2 denotes 3,3'-diiodo-L-thyronine (3,3'-T₂). 3,5T2 denotes 3,5-diiodo-L-thyronine (3,5-T₂). 3',5'T2 denotes 3',5'-diiodo-L-thyronine (3',5'-T₂). T1AA denotes 3-iodothyroacetic acid (3-T₁AA). T1AM denotes 3-iodothyronamine (3-T₁AM). L-T0 denotes L-thyronine (T₀). DL-T0 denotes the racemic form of thyronine.

As shown in Figure 1, among thyroid hormones and their metabolites, the number of HSCs after culture was high when T₄, T₃, rT₃, 3,3'-Diiodo-L-Thyronine (3,3'-T₂), and 3,5-Diiodo-L-thyronine (3,5-T₂) were used. Furthermore, as shown in Figure 2, when these compounds were used, CD48 expression was maintained at low levels. CD48 is known as a marker for cells differentiating from HSCs. Among the compounds, 3,3'-T2 most strongly suppressed CD48 expression.

### <3-2. Long-Term Culture of HSCs>

Method: Thirty mouse HSCs were cultured long-term in medium containing SF-O3, SCF 100ng/mL, TPO 100ng/mL, and 3,3'-T₂ 10µM, hereinafter referred to as mouse T₂ medium. The number of HSCs was recorded at each time point. For the control group mouse HSCs, a medium was used that was mouse T₂ medium with only 3,3'-T₂ removed. The first medium change was performed one week after the start of culture, and thereafter, medium was changed every 4-5 days. During medium changes, 1/2 to 1/4 of the total cells were left behind to ensure the total cell count in the medium did not exceed 3x10⁶ cells/mL. Every 3 to 4 medium changes, differentiated blood cells were removed from the medium. At this time, mouse HSCs were isolated by purifying the cells using a cell sorter. Cultivation was continued long-term as described above.

When calculating HSC numbers, dilution of HSCs during medium changes was taken into account. Specifically, the number of HSCs expanded was calculated by converting the number of HSC clones present in the medium at the time of counting to the number of initial HSCs from which these clones originated, based on the dilution factor. This accounts for the fact that HSC culture involves cell expansion, and the cell count diverges as the culture period lengthens. For example, attempting to continue culturing all expanded cells could potentially require 1L, 2L, or even more medium. However, depending on the intended use of the cultured cells, the number obtainable from tens to hundreds of milliliters of medium may suffice. Therefore, even if culture began with 30 HSCs, after expansion, discarding a portion of the cells allows retaining only the necessary quantity. For instance, during medium change, half the cells could be discarded, continuing culture with only the remaining half. This limits the medium volume used to the amount required for the needed cell count. For example, at a certain point during culture, the situation might be equivalent to culturing "15 initial HSCs from the start of culture," representing a dilution factor of 2. Alternatively, it might be equivalent to culturing "1 initial HSC from the start of culture," representing a dilution factor of 30. As culture progresses further and half the cells are discarded, the situation becomes "cultivating initial HSCs equivalent to 0.5 cells," corresponding to a dilution factor of 60. Repeatedly retaining only a portion of the cells dilutes the number of initial HSCs to a decimal value. This example calculates how many HSCs could be expanded from the original 30 HSCs at the start of culture. For example, suppose 10 HSCs, thought to originate from a single HSC, are detected in the medium after medium replacement. Since the initial number of HSCs was 30, the dilution factor is 30. Therefore, it can be calculated that 30 HSCs expanded to 10x30 = 300 HSCs.

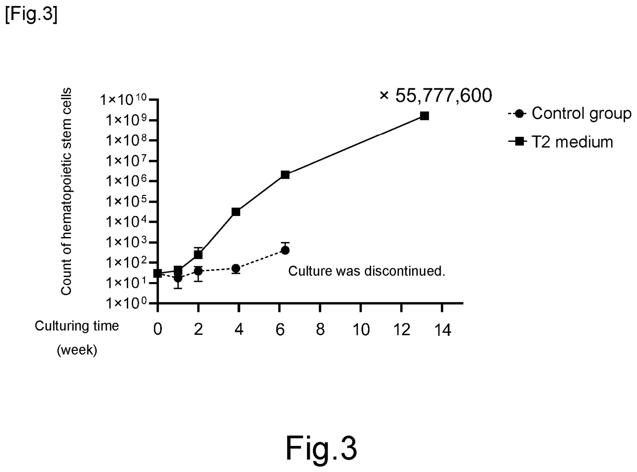

Results: The graph in Figure 3 shows the change in cell number during culture. Each point represents the average result from independent trials. Error bars indicate standard deviation. In the mouse T₂ medium group, the number of HSCs increased rapidly compared to the control group, and this trend continued. That is, the HSC pool expanded, and this trend persisted. In the control group, approximately 99.7% of all cells had died by about 6 weeks after the start of culture. At this point, culture of the control group cells was discontinued. Thereafter, only the cells in the mouse T₂ medium group were continued in culture. Approximately 3 months after the start of culture, the number of cells had increased to about 5x10⁷ times the number of cells at the start of culture.

### <3-3. Functional Evaluation of Cultured HSCs>

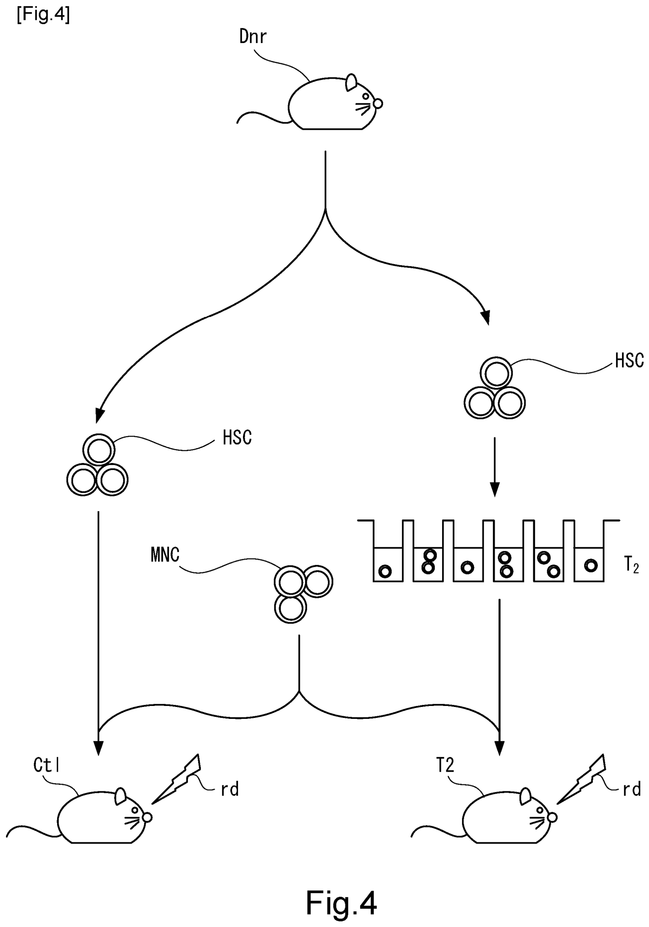

Method: Figure 4 shows an overview of the chimerism experiment. Donor mice (Dnr, Ubc-GFP mice) expressing GFP under the Ubc (Ubiquitin C) promoter were bred. In these donor mice, blood cells other than red blood cells and platelets are Ly5.2-positive. HSCs were collected from the donor mouse and purified. One hundred of these HSCs (GFP⁺HSCs) were cultured for two weeks in Mouse T₂ medium. A recipient mouse (Ly5.1 mouse) irradiated with 8.5 Gy radiation (rd) was prepared separately. In the recipient mouse, all blood cells except red blood cells and platelets are Ly5.1-positive.

All post-culture cells, specifically all cells derived from 100 GFP⁺ HSCs, were transplanted into Ly5.1 mice of the T2 group together with 4x10⁵ Ly5.1 mouse-derived bone marrow mononuclear cells (MNCs) serving as competitor cells. The post-culture cells were expanded in mouse T₂ medium and numbered more than 100. For the control group (Ctl), 100 fresh GFP⁺ HSCs purified by cell sorting were transplanted into Ly5.1 mice along with MNCs. These GFP⁺ HSCs had not been cultured in mouse T₂ medium. After one month post-transplantation, peripheral blood was collected from each recipient mouse. This peripheral blood was then fractionated.

Donor-derived chimerism was calculated based on the frequency of Ly5.2-positive and Ly5.1-positive cells in the peripheral blood. This allowed verification of the efficiency of granulocyte (Gr-1⁺ or Mac-1⁺), B lymphocyte (B220⁺), T lymphocyte (CD4⁺ or CD8⁺), erythrocyte (Ter119⁺), and platelets (CD41⁺) within each recipient mouse. For blood cells other than erythrocytes and platelets in each peripheral blood fraction, donor cell chimerism was calculated using the formula: (Ly5.2⁺ cell count) / {(Ly5.2⁺ cell count) + (Ly5.1⁺ cell count)}. For erythrocytes and platelets, donor-derived cell chimerism was calculated as (GFP⁺ cell count) / {(GFP⁺ cell count) + (GFP⁻ cell count)}.

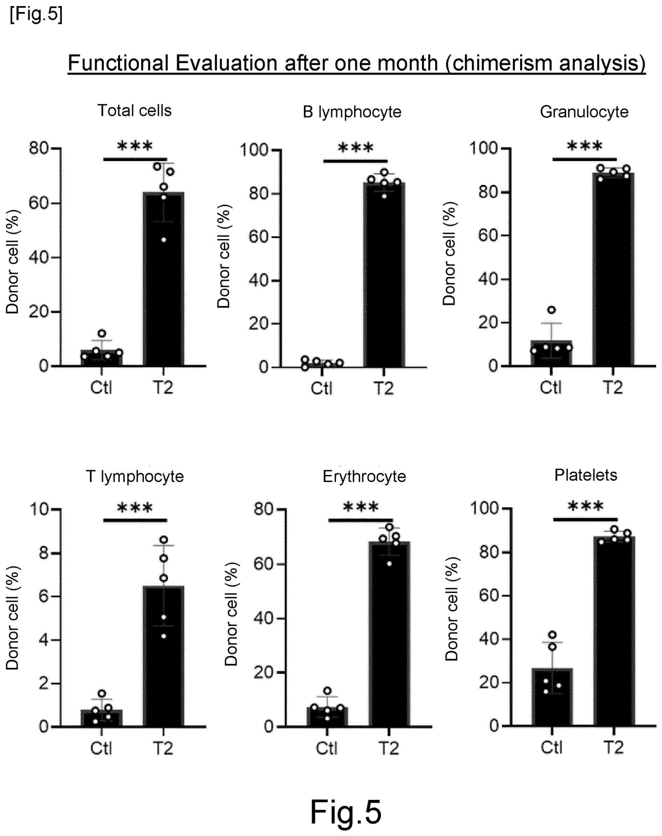

Results: Figure 5 shows the chimerism analysis results. Circles indicate the measured chimerism (%) in each recipient. Error bars indicate standard deviation. *** indicates a P-value below the significance level α=0.001. Compared to the control group (Ctl on the graph's x-axis), the group cultured in mouse T₂ medium (T2 group on the graph's x-axis) showed a predominance of donor mouse HSC progeny over MNC progeny in all blood cell types.

### <3-4. Expansion of Human Umbilical Cord Blood HSCs>

Method: HSCs (CD34⁺ CD38⁻ CD90⁺ CD45RA⁻ EPCR⁺) were obtained by purifying cells from human umbilical cord blood donated by a cord blood bank under ethical approval using a cell sorter. Hereafter, cells with similar surface markers are referred to as human HSCs. Medium containing StemSpan^{®} Serum-Free Expansion Medium (STEMCELL Technologies), human SCF 100ng/mL, human Flt3-ligand 100ng/mL, human TPO 100ng/mL, 10µg/mL human LDL, and UM171 35nM was dispensed into a well plate. One hundred human HSCs were seeded into each well. At this time, 3,3'-T₂ 10µM was added to the medium (T2+ group). Hereafter, this medium containing 3,3'-T₂ is referred to as human T₂ medium. In the control group (T2-), DMSO was added instead of 3,3'-T₂. The control group (T2-) medium contained all the above components, including UM171 at 35 nM. The first medium change was performed one week after the start of culture. Subsequently, 3/4 of the medium was replaced every three days. The number of human HSCs was counted by flow cytometry two weeks after the start of culture.

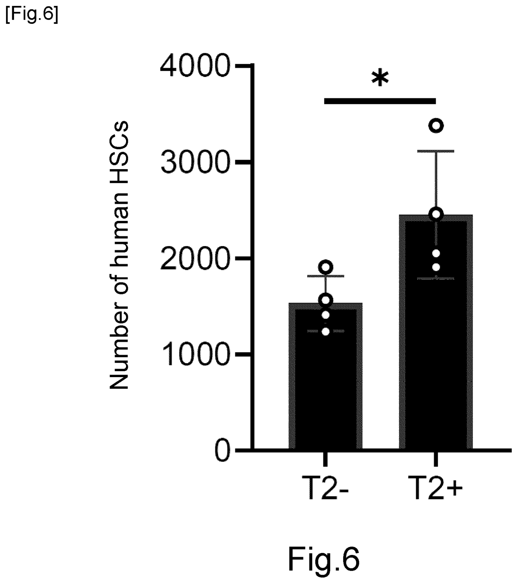

Results: The graph in Figure 6 shows the number of hematopoietic stem cells in each colony. Circles indicate the count for each colony. Error bars indicate standard deviation. * indicates a P-value below the significance level α=0.05. Culturing in human T₂ medium was found to expand human HSCs more efficiently. Furthermore, it was found that the proliferation-promoting effect of thyroid hormone metabolites can be obtained in addition to the proliferation promotion by UM171.

### <3-5. Expansion and Rejuvenation of HSCs Derived from Aged Mice>

Method: Chimeric experiments were performed following the procedures described in Example 3 and shown in Figure 4. An 80-week-old aged mouse was selected as the donor mouse (Dnr). 300 HSCs derived from the aged mouse were used per recipient mouse. HSCs were cultured for 4 weeks in mouse T₂ medium. Peripheral blood was collected from recipient mice one month after HSC transplantation. Donor-derived chimerism for granulocytes (Gr-1⁺ or Mac-1⁺), B lymphocytes (B220⁺), and T lymphocytes (CD4⁺ or CD8⁺) was verified as described in <Experiment 3>.

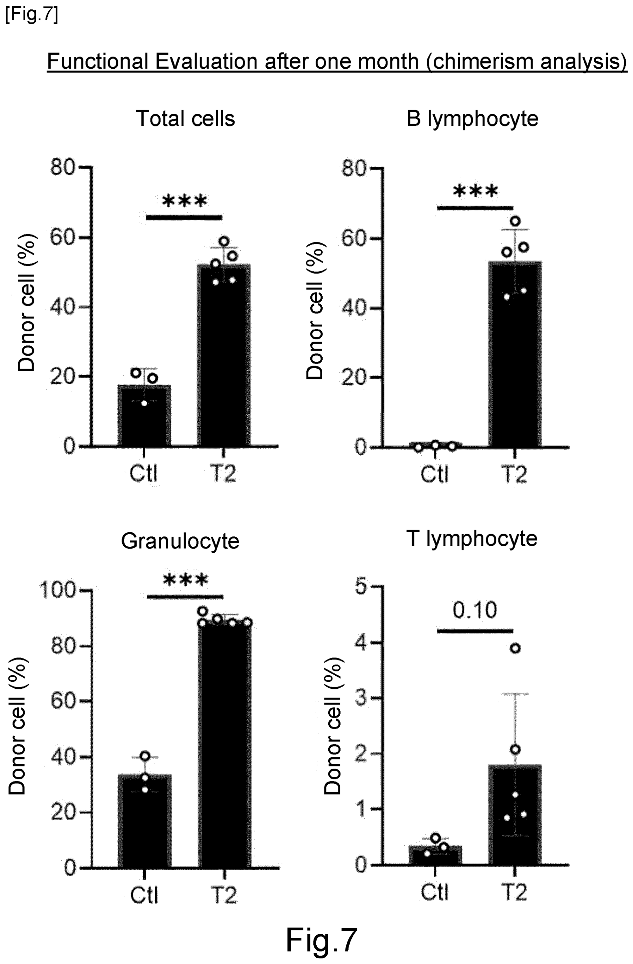

Results: The graph in Figure 7 shows the results of the chimerism analysis. Circles represent the measured chimerism (%) values in each recipient. Error bars indicate standard deviation. *** indicates a P-value below the significance level α=0.001. At one month post-transplantation, the group transplanted with cells cultured in mouse T₂ medium (T2 on the graph's x-axis) showed a predominance of donor mouse HSC progeny over MNC progeny in all blood cell types compared to the control group (Ctl on the graph's x-axis).

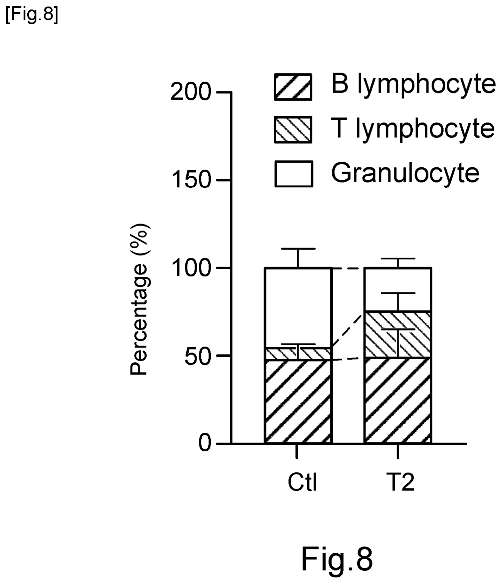

Figure 8 shows the percentage of each blood cell type in mouse blood. Error bars indicate standard deviation. Aged HSCs are characterized by myeloid-biased hematopoiesis. Consequently, in the control (Ctl) group, granulocytes were in competition with lymphocytes, i.e., the sum of T lymphocytes and B lymphocytes. In contrast, in the group transplanted with aged HSCs cultured in mouse T₂ medium, lymphocytes were dominant over granulocytes. Aged HSCs treated with T₂ were shown to exhibit lymphocyte-biased hematopoiesis similar to that of young HSCs. The effect of T₂ treatment was shown to extend beyond simple enhancement of HSC expansion. Specifically, T₂ treatment was shown to functionally rejuvenate the properties of HSCs.

### <3-6. Replacement with Thyroid Hormone Derivative>

Culturing mouse-derived HSCs in medium containing the thyroid hormone derivative sobetirome (manufacturer: MedChem Express, product code: HY-14823) promoted HSC expansion. Additionally, HSC differentiation was suppressed.

### <3-7. Expansion of Mouse-Derived HSCs in Serum-Free Medium>

Mouse-derived HSCs were cultured in serum-free medium containing PVA and T₂ instead of the serum-containing T₂ medium described above. HSC expansion was also promoted in this serum-free medium. Furthermore, HSC differentiation was suppressed. At this time, the optimal concentration of T₂ was 0.1 µM.

### <3-8. Carboxylic Acid Derivatives of Thyronine>

As shown in Figure 1, T1AA, namely 3-iodethyroacetic acid (3-T₁AA), exhibited a tendency to promote cell proliferation. Furthermore, as shown in Figure 2, T1AA exhibited a tendency to reduce CD48 expression. Therefore, the effects of carboxylic acid derivatives of other compounds were confirmed.

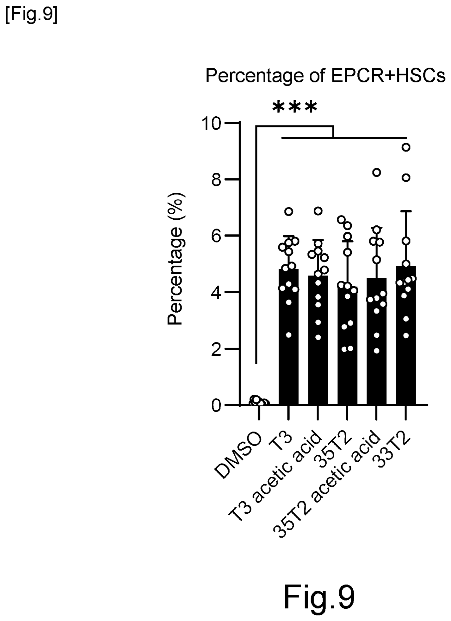

Figure 9 shows a graph of the percentage (%) of EPCR-positive HSCs (EPCR+HSCs) after culturing HSCs in the presence of the carboxylic acid derivatives of thyronine. T3 represents 3,3',5-triiodo-L-thyronine (T₃). T3 acetic acid denotes 3,3',5-triiodo-L-thyroacetic acid (T₃AA). 35T2 denotes 3,5-diiodo-L-thyronine (3,5-T₂). 35T2 acetic acid denotes 3,5-diiodo-L-thyroacetic acid (3,5-T₂AA). 33T2 denotes 3,3'-diiodo-L-thyronine (3,3'-T₂). In each group, the proportion of EPCR+HSCs increased compared to the control group treated with DMSO alone.

### <3-9. Expansion of Other Hematopoietic Lineage Cells>

The effects of the compounds on the expansion of other hematopoietic cells, besides hematopoietic stem cells, were investigated.

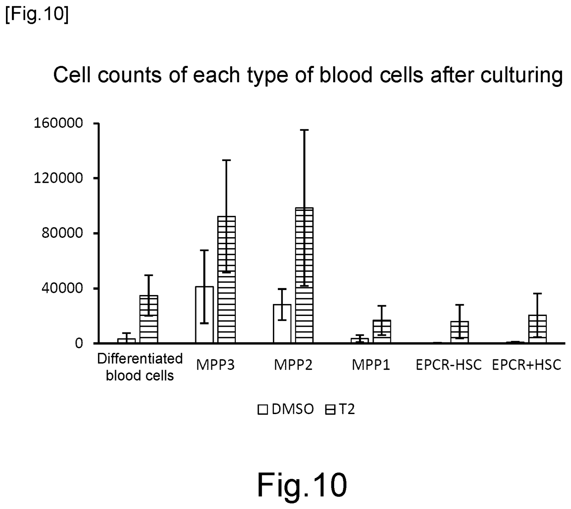

Figure 10 shows the cell counts after 4 weeks of culture of hematopoietic cells in the presence of T2. The control group received only DMSO. Differentiated blood cells are c-Kit-negative cells. MPP3, MPP2, and MPP1 are multipotent progenitor cells (MPPs) referred to as hematopoietic progenitor cells. EPCR-HSC are EPCR-negative HSCs. EPCR-HSC are slightly less functional than EPCR-positive HSCs. EPCR+HSC are EPCR-positive HSCs. EPCR+HSC represent the most undifferentiated population among HSCs.

As shown in Figure 10, in the group cultured with EPCR+HSC in the presence of T2, EPCR+HSC expanded more than in the control group (DMSO). The expansion factor was 204-fold. Furthermore, not only EPCR+HSC but also other cells derived from EPCR+HSC, such as EPCR-HSC, MPP3, MPP2, MPP1, and differentiated blood cells are expanded. The compounds of this embodiment, effective for proliferating hematopoietic stem cells, also contribute to the proliferation of hematopoietic cells other than HSC, such as multipotent progenitor cells and differentiated blood cells. The compounds of this embodiment are effective for expanding EPCR+HSCs, and starting from EPCR+HSCs, increases in EPCR-HSCs, multipotent progenitor cells (e.g., MPP3, MPP2, MPP1), and differentiated blood cells are also observed similarly to normal hematopoiesis. This demonstrates that a method for promoting the expansion of hematopoietic stem cells has been established.

The present invention is not limited to the above and may be appropriately modified within the scope of the spirit and scope of the invention. This application claims priority based on Japanese Patent Application No. 2023-102048 filed on June 21, 2023, and incorporates its entire disclosure herein by reference.

### Symbol Explanation

"Ctl": Control group, "Dnr": Donor, "HSC": Hematopoietic stem cell, "MNC": (Bone marrow) Mononuclear cell, 'T2': Group of cells cultured in mouse T2 medium, "rd": Radiation

## Claims

1. A culture medium for expanding hematopoietic cells capable of cell division, comprising a compound (I) of the following formula or a pharmaceutically acceptable salt thereof: 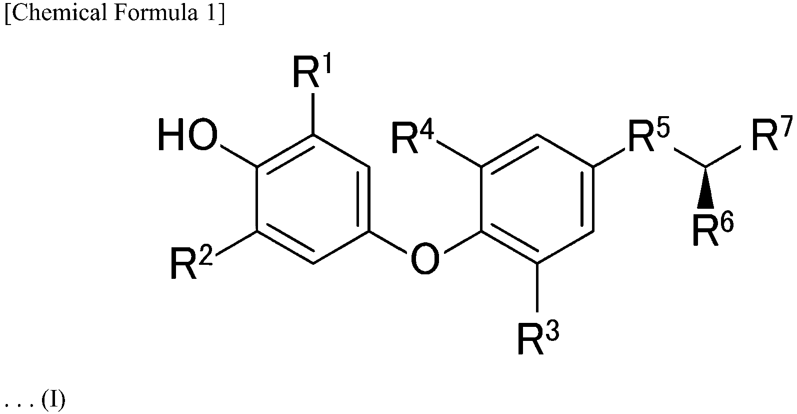 in the formula,
R¹, R², and R³ are each hydrogen, straight-chain or branched C₁-C₄ alkyl, bromo, or iodo; R⁴ is straight-chain or branched C₁-C₄ alkyl, bromo, or iodo,
R⁵ is oxygen, methylene, or a direct bond,
R⁶ is hydrogen or -NH₂,
R⁷ is hydrogen or -COOH, provided that R⁶ and R⁷ are not both hydrogen.

2. The medium according to Claim 1, wherein
(1) R⁵ is oxygen or methylene,
R⁶ is hydrogen or -NH₂,
R⁷ is hydrogen or -COOH, provided that R⁶ and R⁷ are not both hydrogen,
or
(2) R⁵ is a direct bond,
R⁶ is hydrogen,
R⁷ is -COOH.

3. The medium according to Claim 1, wherein,
Compound (I) is any one of: 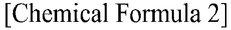 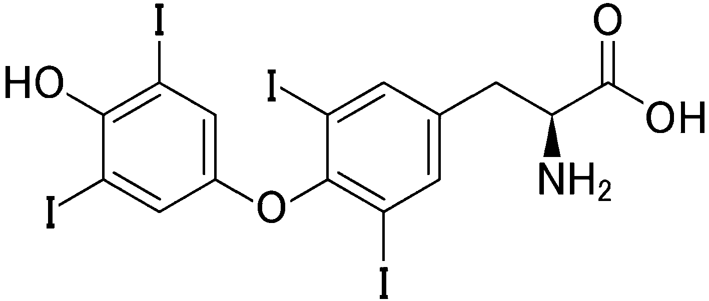
thyroxine (T₄), 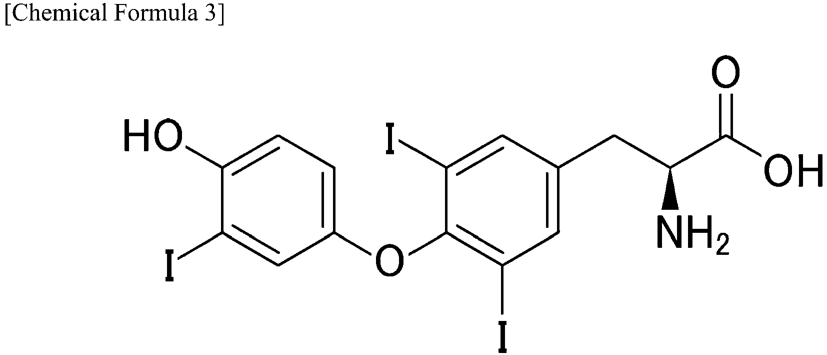
3,3',5-triiodo-L-thyronine (T₃), 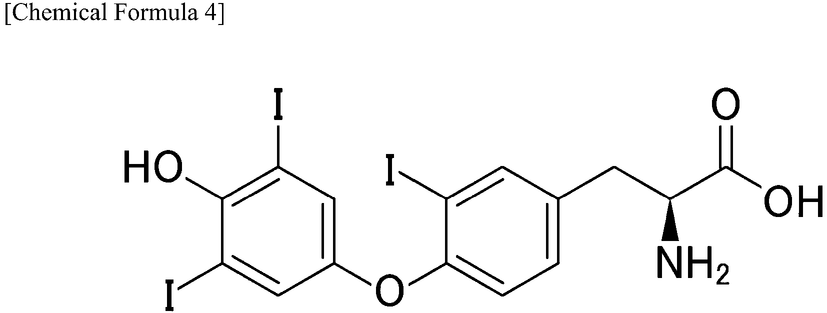
3,3',5'-Triiodothyronine (rT₃), 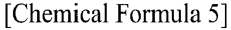 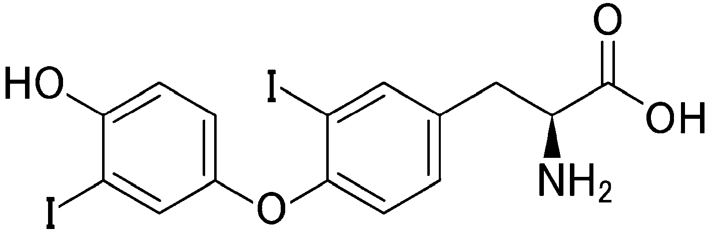
3,3'-Diiodo-L-thyronine (3,3'-T₂), 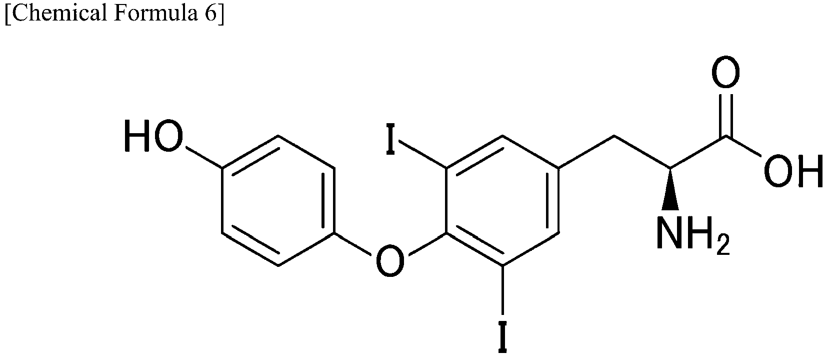
3,5-Diiodo-L-thyronine (3,5-T₂), 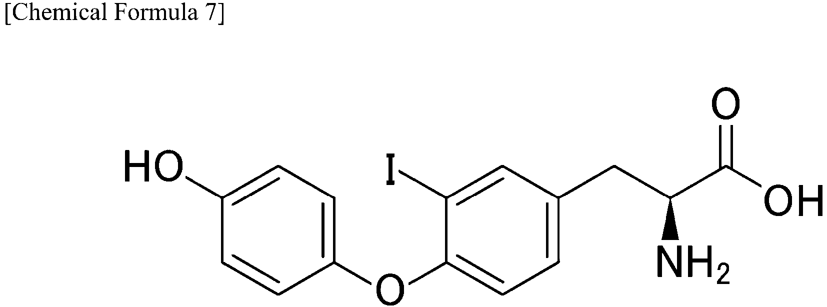
3-Iodo-L-thyronine (3-T₁), 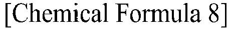 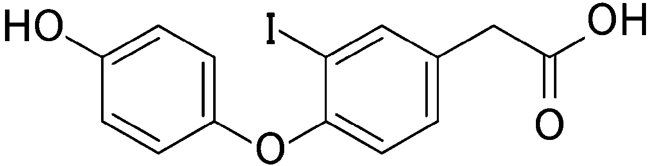
3-Iodo-L-thyroacetic acid (3-T₁AA), 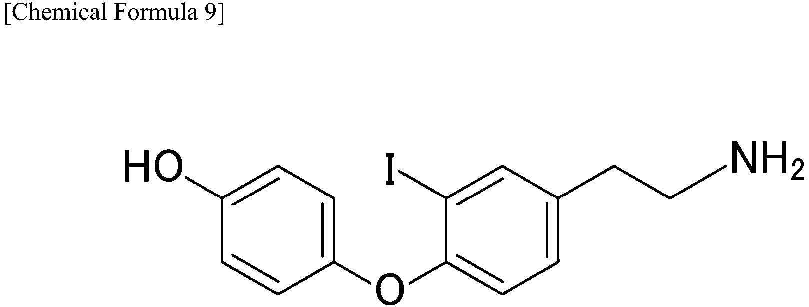
3-Iodo-L-thyronamine (3-T₁AM), 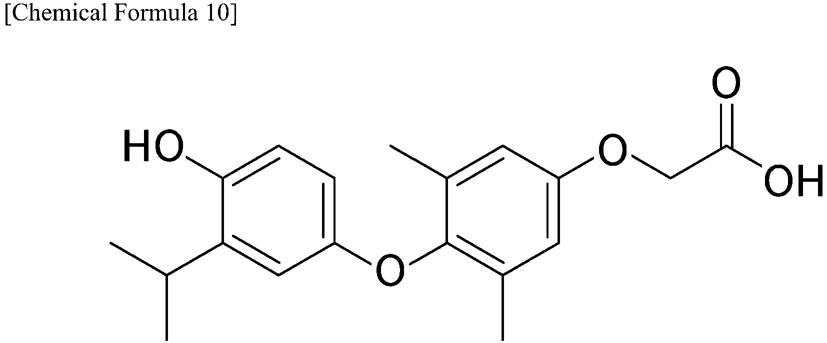
Sobetirome, 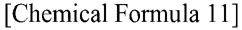 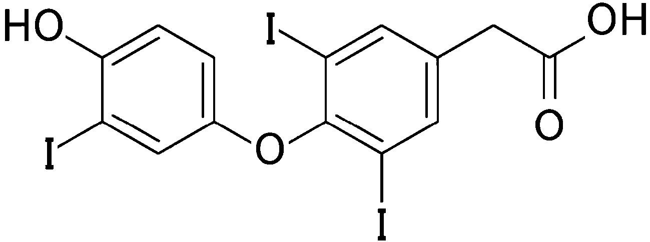
3,3',5-Triiodo-L-thyroacetic acid (3,3',5-T₃AA) and 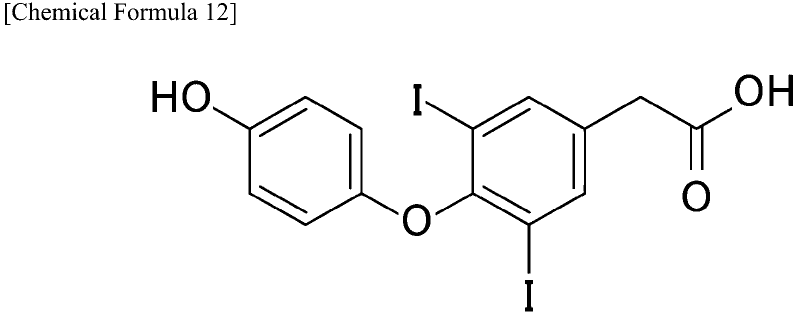
3,5-Diiodo-L-thyroacetic acid (3,5-T₂AA).

4. The medium according to Claim 1, wherein
R¹ is hydrogen,
R² and R³ are such that one is hydrogen, straight-chain or branched C₁-C₄ alkyl, bromo, or iodo, and the other is hydrogen.

5. The medium according to Claim 1, wherein compound (I) is either 3,5-diiodo-L-thyronine (3,5-T₂) or 3,3'-diiodo-L-thyronine (3,3'-T₂).

6. The medium according to Claim 1, wherein
the hematopoietic cells are obtained from an aged donor, or
the hematopoietic cells are obtained from umbilical cord blood.

7. The medium according to Claim 1, for expanding the hematopoietic cells without mixing them with hematopoietic cells at other differentiation stages.

8. The medium according to Claim 1, for expanding the hematopoietic cells and further expanding differentiated hematopoietic cells generated during the process of expanding the hematopoietic cells.

9. The medium according to Claim 1, wherein
the aforementioned hematopoietic cells are any one of:
hematopoietic stem cells that have not yet differentiated into multipotent progenitor cells, multipotent progenitor cells that have completed differentiation from hematopoietic stem cells but have not yet differentiated into precursor cells of differentiated blood cells, and differentiated blood cells that have completed differentiation from their precursor cells.

10. The medium according to Claim 1, wherein
the medium is serum-free and comprises compound (I), or the medium is a serum medium and comprises compound (I) at 0.1 µM to 1 mM.

11. A method for producing a clone of hematopoietic cells comprising expanding hematopoietic cells capable of cell division in vitro while applying compound (I) of the following formula or a pharmaceutically acceptable salt thereof to said hematopoietic cells: 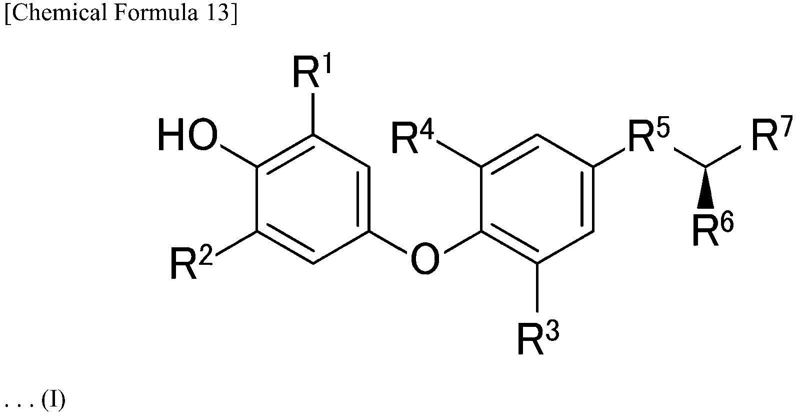 wherein
R¹, R², and R³ are each hydrogen, straight-chain or branched C₁-C₄ alkyl, bromo, or iodo;
R⁴ is straight-chain or branched C₁-C₄ alkyl, bromo, or iodo,
R⁵ is oxygen, methylene, or a direct bond,
R⁶ is hydrogen or -NH₂,
R⁷ is hydrogen or -COOH, provided that R⁶ and R⁷ are not both hydrogen.

12. A clone of hematopoietic cells obtained by applying compound (I) of the formula below or a pharmaceutically acceptable salt thereof when expanding hematopoietic cells capable of cell division in vitro, wherein: 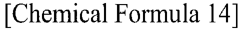 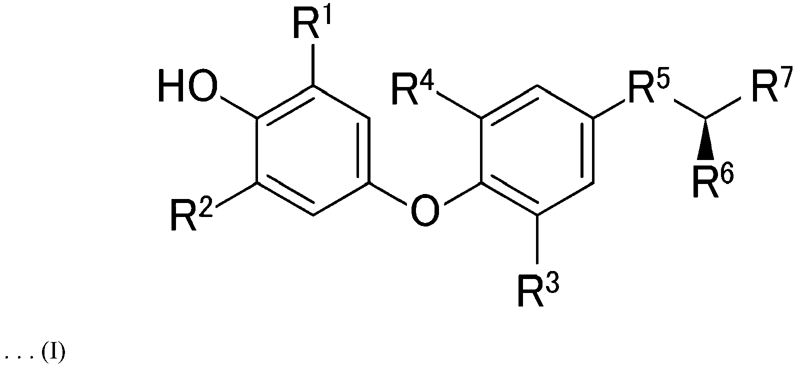 wherein
R¹, R², and R³ are each hydrogen, straight-chain or branched C₁-C₄ alkyl, bromo, or iodo and,
R⁴ is straight-chain or branched C₁-C₄ alkyl, bromo, or iodo,
R⁵ is oxygen, methylene, or a direct bond,
R⁶ is hydrogen or -NH₂,
R⁷ is hydrogen or -COOH, provided that R⁶ and R⁷ are not both hydrogen,
the seeded hematopoietic cells are obtained from an aged donor.

13. An agent for adding to serum medium or serum-free medium to expand hematopoietic cells capable of cell division in vitro,
comprising as an active ingredient a compound (I) of the following formula or a pharmaceutically acceptable salt thereof: 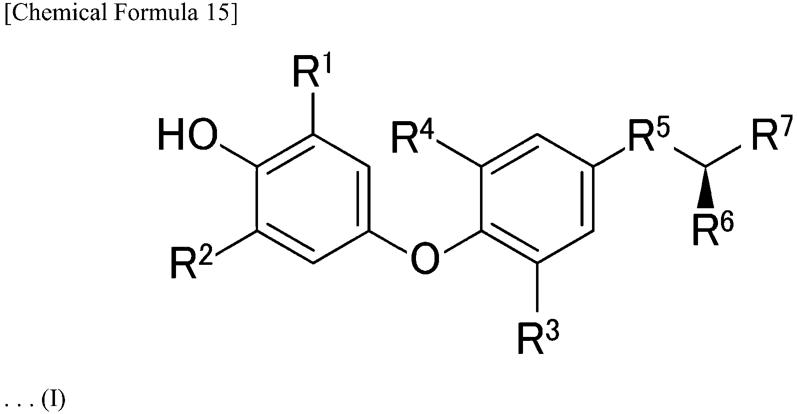
wherein
R¹, R², and R³ are each hydrogen, straight-chain or branched C₁-C₄ alkyl, bromo, or iodo;
R⁴ is straight-chain or branched C₁-C₄ alkyl, bromo, or iodo,
R⁵ is oxygen, methylene, or a direct bond,
R⁶ is hydrogen or -NH₂,
R⁷ is hydrogen or -COOH, provided that R⁶ and R⁷ are not both hydrogen.
